# EUROPEAN PATENT APPLICATION

(11) **EP 4 212 165 A1**
(43) Date of publication of application: **19.07.2023**
(21) Application number: 21828789.4
(22) Date of filing: 21.06.2021
(51) Int. Cl.: A61K 35/747, A23C 9/123, A23L 33/135, A61K 35/20, A61P 29/00, A61P 43/00, C12N 1/20

(54) **COMPOSITION FOR PROMOTING PRODUCTION OF INTERLEUKIN-10**

(30) Priority: 22.06.2020 JP 2020107035
(71) Applicant: MEIJI CO., LTD, Chuo-ku Tokyo 104-8306 (JP)
(72) Inventor: TOSHIMITSU, Takayuki, Hachioji-shi, Tokyo 192-0919 (JP); KAWAI, Yoshitaka, Hachioji-shi, Tokyo 192-0919 (JP)
(74) Representative: TBK
(86) International application number: PCT/JP2021/023326
(87) International publication number: WO 2021/261423

(57) **Abstract**

The present invention relates to a composition for promoting the production of interleukin-10, the composition including: a lactic acid bacterium belonging to the genus *Lactobacillus;* and a fermented milk.

## Description

### CROSS-REFERENCE TO RELATED APPLICATIONS

This application is based upon and claims the benefit of priority from Japanese Patent Application No. 2020-107035 filed June 22, 2020; the entire contents of which are incorporated herein by reference.

### BACKGROUND OF THE INVENTION

### Field of the Invention

The present invention relates to a composition for promoting the production of interleukin-10.

### Background Art

Interleukin-10 (IL-10) is an anti-inflammatory cytokine produced by immune cells (such as dendritic cells and macrophages), and is responsible for inhibiting an excessive inflammation in a living body. That is, inducing the production of IL-10 enables to ameliorate not only inflammation, but also diseases caused by inflammation, and the like. For example, it has been reported that it is possible to inhibit enteritis in a subject by administering a lactic acid bacterium having a high IL-10 production inducing activity to the subject (for example, Non-patent Documents 1 and 2). It has also been reported that myocardial infarction can be reduced trough inhibiting inflammation by overexpressing IL-10 by genetic recombination (for example, Non-patent Document 3). Further, it has been reported that it is possible to prevent and/or treat a disease caused by inflammation in a subject, by administering a lactic acid bacterium that promotes the production of IL-10 to the subject (for example, Patent Document 1, Non-patent Document 4). Therefore, the amount of IL-10 in a living body has been regarded as an important index of the inhibition of inflammation.

On the other hand, interleukin-12 (IL-12) is known to be different from IL-10 in that it has a function to promote inflammation in a living body, although being the same as IL-10 in that it is a cytokine produced by immune cells (for example, Non-patent Documents 5 and 6). Therefore, the amount of IL-12 in a living body has been regarded as one of the indices of the promotion of inflammation.

Accordingly, the ratio of IL-10 with respect to IL-12 (namely, the amount of IL-10/the amount of IL-12) has been regarded as an important index of the inflammation inhibitory activity (anti-inflammatory activity), as with the amount of IL-10 (for example, Non-patent Documents 7 to 9).

Conventionally, search for bacterial strains (such as lactic acid bacteria) having a high IL-10 production inducing activity and bacterial strains having a low IL-12 production inducing activity have been performed, and lactic acid bacterial strains have been reported that promote the production of IL-10 and/or inhibit the production of IL-12 (for example, Patent Document 2 and Non-patent Document 10). However, since inflammation suggests connections with various kinds of diseases (such as metabolic syndrome, dyslipidemia, diabetes, obesity, hyperglycemia, cancers and autoimmune diseases), there are demands for a method that promotes the production of IL-10 more efficiently than ever and/or inhibits the production of IL-12, and a method that eventually ameliorate inflammation further efficiently.

### Prior Art Documents

### Patent Documents

Patent Document 1: WO 2012/014971
Patent Document 2: WO 2018/038004

### Non-patent Documents

Non-patent Document 1: O'Mahony L. et al. "Lactobacillus and bifidobacterium in irritable bowel syndrome: symptom responses and relationship to cytokine profiles." Gastroenterology, 2005; 128 (3): 541 to 51
Non-patent Document 2: Sokol H. et al. "Faecalibacterium prausnitzii is an anti-inflammatory commensal bacterium identified by gut microbiota analysis of Crohn disease patients." Proc. Natl. Sci. U.S.A., 2008; 105 (43): 16731 to 16736
Non-patent Document 3: Yang Yu et al. "Central gene transfer of interleukin-10 reduces hypothalamic inflammation and evidence of heart failure in rats after myocardial infarction." Circ. Res., 2007; 101 (3): 304 to 312
Non-patent Document 4: Toshimitsu T. et al. "Identification of lactobacillus plantarum strain that ameliorates chronic inflammation and metabolic disorders in obese and type 2 diabetic mice." J. Dairy Sci., 2016; 99 (2): 933 to 946
Non-patent Document 5: Sennello JA, et al. Interleukin-18, together with interleukin-12, induces severe acute pancreatitis in obese but not in nonobese leptin-deficient mice." Proc Natl Acad Sci U S A 105: 8085 to 8090, 2008
Non-patent Document 6: Pini M, et al. "Effect of dietinduced obesity on acute pancreatitis induced by administration of interleukin-12 plus interleukin-18 in mice." Obesity 18:476 to 481, 2010
Non-patent Document 7: Toshimitsu T, et al. "Effects of Lactobacillus plantarum strain OLL2712 culture conditions on the anti-inflammatory activities for murine immune cells and obese and type 2 diabetic mice." Appl. Environ. Microbiol. 83, pii: e03001 to 03016, 2017
Non-patent Document 8: Foligne B, et al. "Correlation between in vitro and in vivo immunomodulatory properties of lactic acid bacteria." World J Gastroenterol 13: 236 to 243, 2007
Non-patent Document 9: Sokol H, et al. "Faecalibacterium prausnitzii is an anti-inflammatory commensal bacterium identified by gut microbiota analysis of Crohn disease patients." Proc Natl Acad Sci U S A 105: 16731 to 16736, 2008
Non-patent Document 10: Toshimitsu T. et al. "Effects of 12-wk Lactobacillus plantarum OLL2712 treatment on glucose metabolism and chronic inflammation in prediabetic individuals: A single-arm pilot study." Nutrition, 2019; 58: 175 to 180

### SUMMARY OF THE INVENTION

An object of the present invention is to provide a composition useful in promoting the production of IL-10 in immune cells. It is also an object of the present invention to provide a composition useful in inhibiting the production of IL-12 in immune cells.

As a result of intensive studies, the present inventors have found that, when a lactic acid bacterium belonging to the genus *Lactobacillus* and a fermented milk are administered to a subject in combination, it is possible to promote the production of IL-10, and further, to inhibit the production of IL-12, in immune cells such as bone marrow-derived dendritic cells of the subject. The present invention is based on such a finding.

According to the present invention, the following inventions are provided.
[1] A composition for promoting the production of interleukin-10, the composition including: a lactic acid bacterium belonging to the genus *Lactobacillus;* and a fermented milk.
[2] The composition according to [1], wherein the composition is for inhibiting the production of interleukin-12.
[3] The composition according to [1] or [2], wherein the composition is for increasing the ratio of the amount of interleukin-10 with respect to the amount of interleukin-12.
[4] The composition according to any one of [1] to [3], wherein the composition is an anti-inflammatory composition.
[5] The composition according to any one of [1] to [4], wherein the composition is for treating or preventing a disease caused by inflammation.
[6] The composition according to [5], wherein the disease caused by inflammation is selected from the group consisting of metabolic syndrome, dyslipidemia, diabetes, obesity, hyperglycemia, cancers and autoimmune diseases.
[7] The composition according to any one of [1] to [6], wherein the lactic acid bacterium is *Lactobacillus plantarum.*
[8] The composition according to any one of [1] to [7], wherein the lactic acid bacterium contains a dead bacterial cell of the lactic acid bacterium.
[9] The composition according to any one of [1] to [8], wherein the lactic acid bacterium contains a heat-treated dead bacterial cell.
[10] The composition according to any one of [1] to [9], wherein the composition is a food composition.
[11] The composition according to any one of [1] to [9], wherein the composition is a pharmaceutical composition.
[12] The composition according to any one of [1] to [11], wherein the lactic acid bacterium has a 16S rRNA gene having a homology of 90% or more to the nucleotide sequence represented by SEQ ID NO: 1.
[13] The composition according to any one of [1] to [12], wherein the lactic acid bacterium is *Lactobacillus plantarum* strain OLL2712 deposited under the accession number FERM BP-11262.
[14] The composition according to any one of [1] to [13], wherein the fermented milk has an average particle size of from 0.5 to 100 µm.
[15] A method of producing the composition according to any one of [1] to [14], the production method including:
   a starter inoculation step of inoculating a raw material milk with a starter to obtain a fermented milk base material;
   a fermentation step of fermenting the fermented milk base material to obtain a fermented milk;
   a lactic acid bacterium addition step of adding a lactic acid bacterium belonging to the genus Lactobacillus to at least one selected from the group consisting of the raw material milk, the fermented milk base material and the fermented milk, to obtain a lactic acid bacterium-containing composition; and
   a stirring step of stirring at least one selected from the group consisting of the raw material milk, the fermented milk base material, the fermented milk and the lactic acid bacterium-containing composition.
[16] The production method according to [15], wherein the stirring in the stirring step is carried out under such conditions that the average particle size of the fermented milk containing the lactic acid bacterium belonging to the genus *Lactobacillus* is adjusted within the range of from 0.5 to 100 µm.
[17] The production method according to [15] or [16], wherein the stirring in the stirring step is carried out under the condition of from 30 to 500 rpm.
[18] A use of the composition according to any one of [1] to [14] for the production of a pharmaceutical product for treating a disease caused by a decrease in the amount of production of interleukin-10.
[19] The use according to [18], wherein the disease is selected from the group consisting of inflammation and a disease caused by inflammation.
[20] The use according to [19], wherein the disease caused by inflammation is selected from the group consisting of metabolic syndrome, dyslipidemia, diabetes, obesity, hyperglycemia, cancers and autoimmune diseases.
[21] A use of the composition according to any one of [1] to [14] for the production of a food or beverage product for treating a disease caused by a decrease in the amount of production of interleukin-10.
[22] The use according to [21], wherein the disease is selected from the group consisting of inflammation and a disease caused by inflammation.
[23] The use according to [21], wherein the disease caused by inflammation is selected from the group consisting of metabolic syndrome, dyslipidemia, diabetes, obesity, hyperglycemia, cancers and autoimmune diseases.
[24] A use of the composition according to any one of [1] to [14] for promoting the production of interleukin-10.
[25] A method of promoting the production of interleukin-10, the method comprising allowing a subject in need thereof to ingest an effective amount of the composition according to any one of [1] to [14].

According to the present invention, it is possible to promote the production of IL-10 known to inhibit inflammation and to inhibit the production of IL-12 known to promote inflammation, in immune cells of a subject, by administering to the subject a lactic acid bacterium belonging to the genus *Lactobacillus* in combination with a fermented milk. Therefore, the present invention enables to efficiently inhibit inflammation.

### BRIEF DESCRIPTION OF THE DRAWINGS

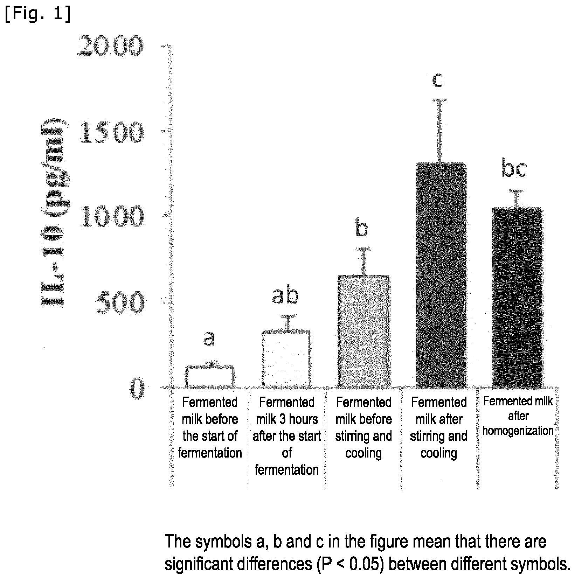

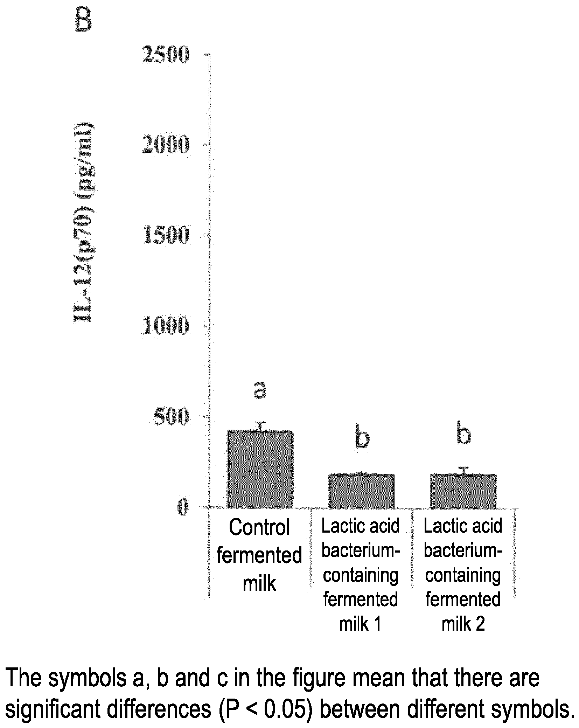

FIG. 1 is a graph showing the results of a multiple comparison test using the Tukey-Kramer method on the IL-10 production promoting activity of the composition according to the present invention, analyzed at each of the time points (before the start of fermentation, 3 hours after the start of fermentation, before stirring and cooling, after stirring and cooling, and after homogenization) in the production process of the composition.

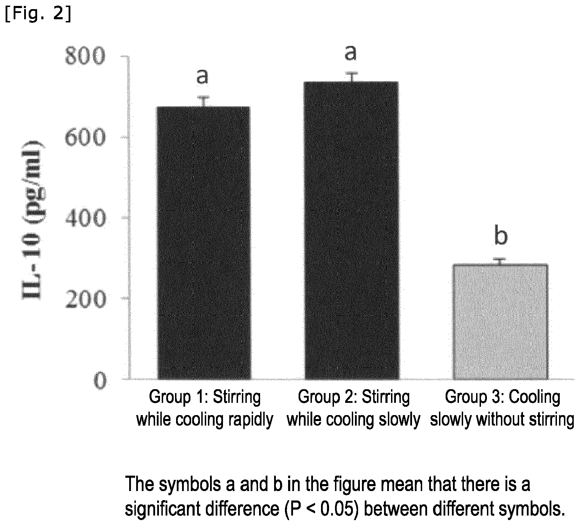

FIG. 2 is a graph showing the results of a multiple comparison test using the Tukey-Kramer method on the IL-10 production promoting activity of the composition, analyzed for each cooling/stirring condition of the fermented milk.

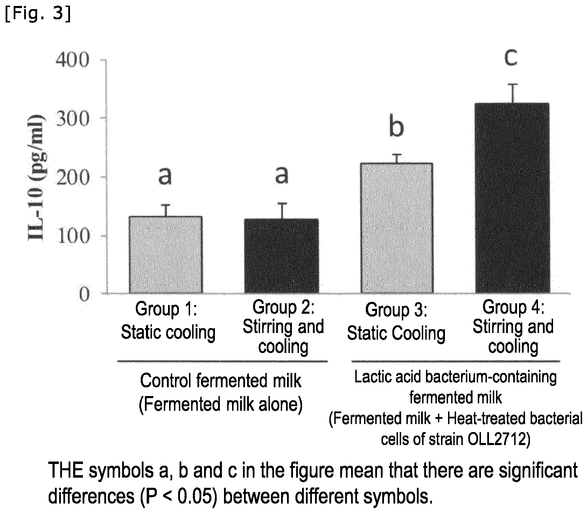

FIG. 3 is a graph showing the results of a multiple comparison test using the Tukey-Kramer method on the IL-10 production promoting activity of the composition, analyzed for each cooling/stirring condition of the fermented milk containing the heat-treated bacterial cells of the genus *Lactobacillus* lactic acid bacterium strain OLL2712, or of the fermented milk which does not contain the heat-treated bacterial cells of the genus *Lactobacillus* lactic acid bacterium strain OLL2712.

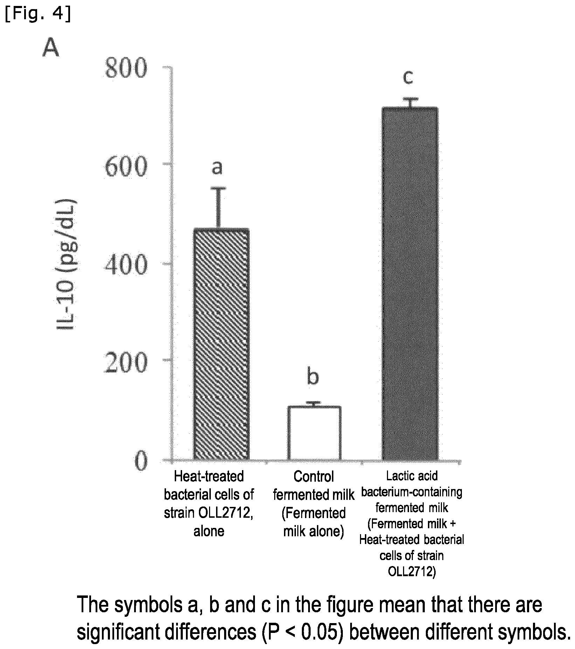

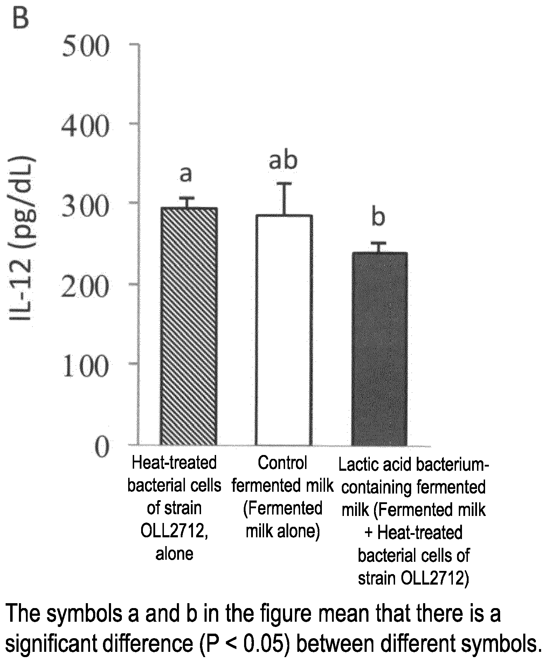

FIG. 4A is a graph showing the results of a multiple comparison test using the Tukey-Kramer method on the IL-10 production promoting activity in bone marrow-derived dendritic cells, analyzed for each of: the heat-treated bacterial cells of the genus *Lactobacillus* lactic acid bacterium strain OLL2712; the fermented milk (control fermented milk); and the combination (lactic acid bacterium-containing fermented milk) of the fermented milk and the heat-treated bacterial cells of the strain OLL2712. FIG. 4B is a graph showing the results of a multiple comparison test using the Tukey-Kramer method on the IL-12 production inhibitory activity in bone marrow-derived dendritic cells, analyzed for each of: the heat-treated bacterial cells of the strain OLL2712; the fermented milk (control fermented milk); and the combination (lactic acid bacterium-containing fermented milk) of the fermented milk and the heat-treated bacterial cells of the strain OLL2712.

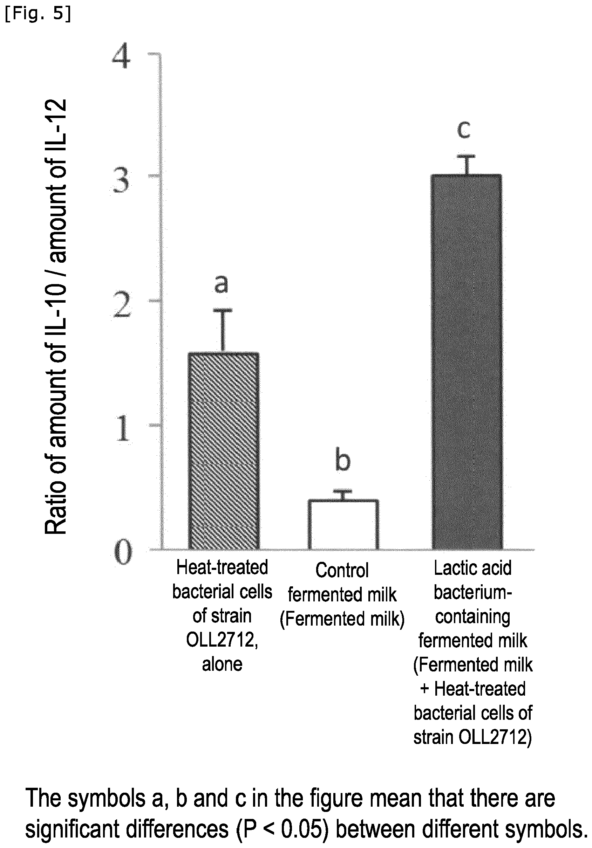

FIG. 5 is a graph showing the results of a multiple comparison test using the Tukey-Kramer method on the ratio (the amount of IL-10/the amount of IL-12) of the amount of IL-10 with respect to the amount of IL-12 in bone marrow-derived dendritic cells, analyzed for each of: the heat-treated bacterial cells of the strain OLL2712; the fermented milk (control fermented milk); and the combination (lactic acid bacterium-containing fermented milk) of the fermented milk and the heat-treated bacterial cells of the strain OLL2712.

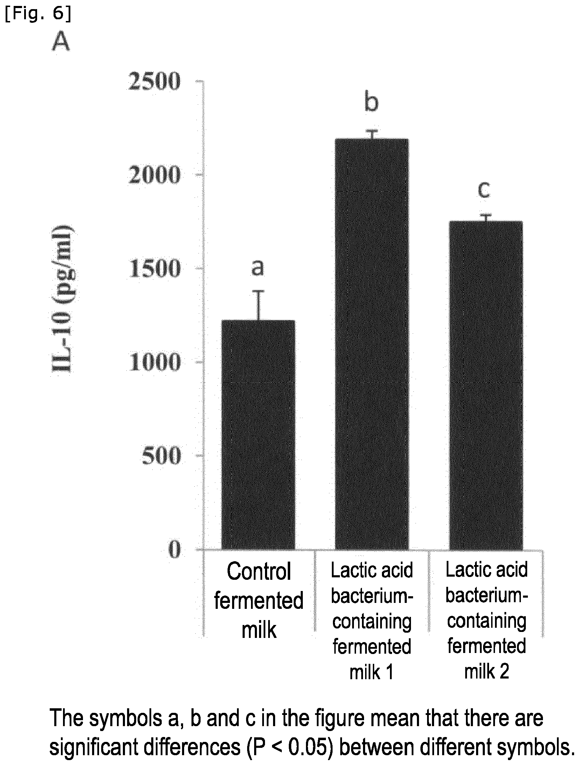

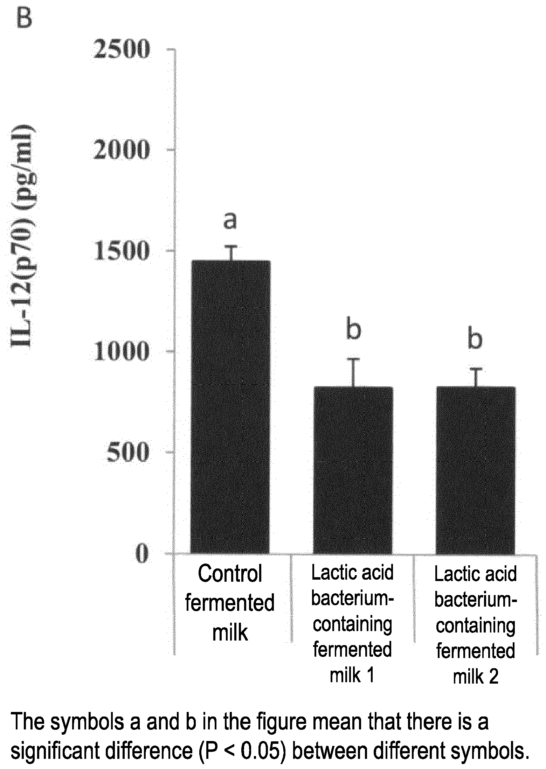

FIG. 6A is a graph showing the results of a multiple comparison test using the Tukey-Kramer method on the IL-10 production promoting activity in bone marrow-derived dendritic cells, analyzed for each of: the fermented milk (control fermented milk) prepared using a starter A; the combination (lactic acid bacterium-containing fermented milk 1) of the fermented milk prepared using the starter A, and the heat-treated bacterial cells of the strain OLL2712; and the combination (lactic acid bacterium-containing fermented milk 2) of the fermented milk prepared using the starter A, and the heat-treated bacterial cells of the strain P200021. FIG. 6B is a graph showing the results of a multiple comparison test using the Tukey-Kramer method on the IL-12 production inhibitory activity in bone marrow-derived dendritic cells, analyzed for each of: the fermented milk (control fermented milk) prepared using a starter A; the combination (lactic acid bacterium-containing fermented milk 1) of the fermented milk prepared using the starter A, and the heat-treated bacterial cells of the strain OLL2712; and the combination (lactic acid bacterium-containing fermented milk 2) of the fermented milk prepared using the starter A, and the heat-treated bacterial cells of the strain P200021.

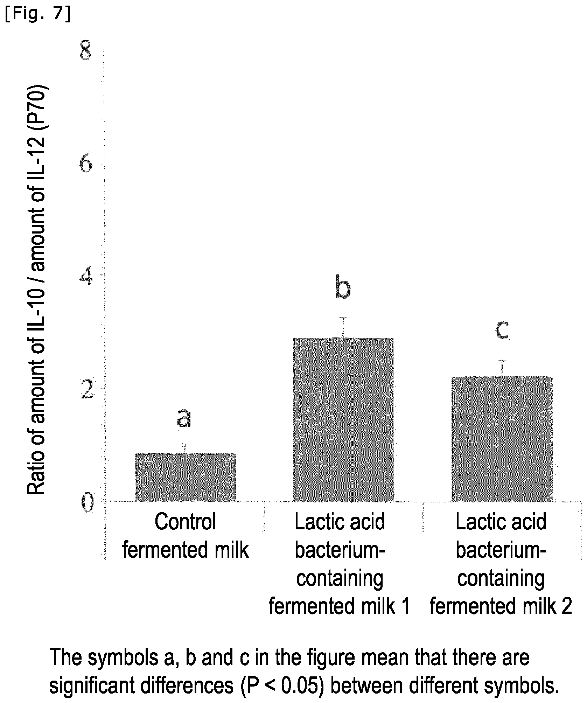

FIG. 7 is a graph showing the results of a multiple comparison test using the Tukey-Kramer method on the ratio (the amount of IL-10/the amount of IL-12) of the amount of IL-10 with respect to the amount of IL-12 in bone marrow-derived dendritic cells, analyzed for each of: the fermented milk (control fermented milk) prepared using a starter A; the combination (lactic acid bacterium-containing fermented milk 1) of the fermented milk prepared using the starter A, and the heat-treated bacterial cells of the strain OLL2712; and the combination (lactic acid bacterium-containing fermented milk 2) of the fermented milk prepared using the starter A, and the heat-treated bacterial cells of the strain P200021.

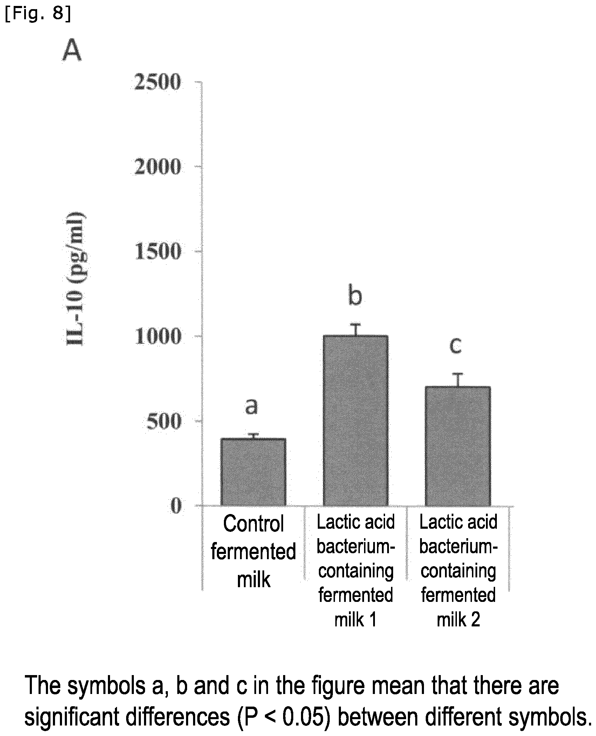

FIG. 8A is a graph showing the results of a multiple comparison test using the Tukey-Kramer method on the IL-10 production promoting activity in bone marrow-derived dendritic cells, analyzed for each of: the fermented milk (control fermented milk) prepared using a starter B; the combination (lactic acid bacterium-containing fermented milk 1) of the fermented milk prepared using the starter B, and the heat-treated bacterial cells of the strain OLL2712; and
   the combination (lactic acid bacterium-containing fermented milk 2) of the fermented milk prepared using the starter B, and the heat-treated bacterial cells of the strain P200021. FIG. 8B is a graph showing the results of a multiple comparison test using the Tukey-Kramer method on the IL-12 production inhibitory activity in bone marrow-derived dendritic cells, analyzed for each of: the fermented milk (control fermented milk) prepared using a starter B; the combination (lactic acid bacterium-containing fermented milk 1) of the fermented milk prepared using the starter B, and the heat-treated bacterial cells of the strain OLL2712; and the combination (lactic acid bacterium-containing fermented milk 2) of the fermented milk prepared using the starter B, and the heat-treated bacterial cells of the strain P200021.

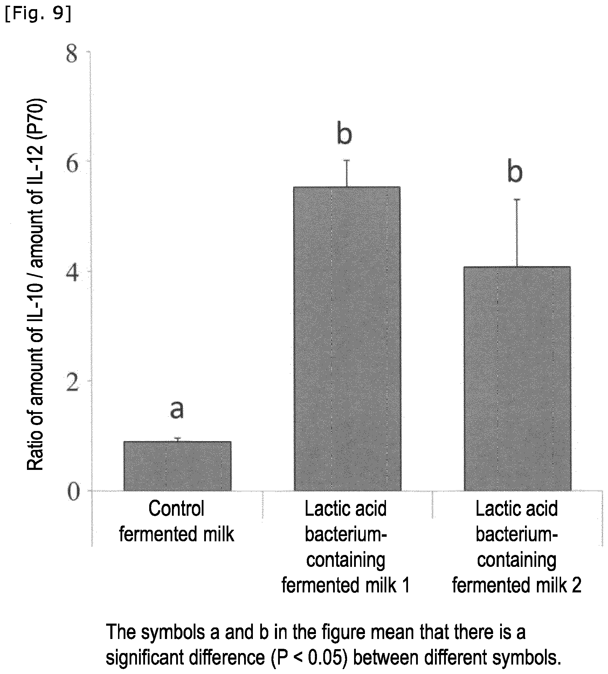

FIG. 9 is a graph showing the results of a multiple comparison test using the Tukey-Kramer method on the ratio (the amount of IL-10/the amount of IL-12) of the amount of IL-10 with respect to the amount of IL-12 in bone marrow-derived dendritic cells, analyzed for each of: the fermented milk (control fermented milk) prepared using a starter B; the combination (lactic acid bacterium-containing fermented milk 1) of the fermented milk prepared using the starter B, and the heat-treated bacterial cells of the strain OLL2712; and the combination (lactic acid bacterium-containing fermented milk 2) of the fermented milk prepared using the starter B, and the heat-treated bacterial cells of the strain P200021.

### DETAILED DESCRIPTION OF THE INVENTION

### Composition For Promoting Production of Interleukin-10

One aspect of the present invention provides a composition for promoting the production of interleukin-10 (IL-10) of a subject, the composition containing a lactic acid bacterium belonging to the genus *Lactobacillus* (hereinafter, also referred to as "genus *Lactobacillus* lactic acid bacterium" or "lactic acid bacterium") and a fermented milk. In the present specification, the expressions "the production of IL-10 of a subject", "the production of IL-10 in a subject" and the like each refer to "the production of IL-10 in immune cells of a subject". In the present specification, the "immune cells" are not particularly limited as long as the cells have an ability to produce IL-10, and examples thereof include bone marrow-derived dendritic cells (BMDCs).

According to one embodiment of the present invention, the subject to whom the composition of the present invention is applied is not particularly limited as long as the effects of the present invention are exhibited. The subject may be, for example, a mammal such as a human, a monkey, a chimpanzee, a cow, a horse, a sheep or a goat, and is preferably a human.

The composition according to the present invention is capable of promoting the production of IL-10 in immune cells of a subject to whom the composition is applied (hereinafter, also referred to as "subject to be applied") by combining a genus *Lactobacillus* lactic acid bacterium with a fermented milk, as compared to the case of administering the genus *Lactobacillus* lactic acid bacterium or the fermented milk singly. It is an unexpected fact to those skilled in the art that the combination of a genus *Lactobacillus* lactic acid bacterium and a fermented milk enables to efficiently promote the production of IL-10 in a subject as described above.

Further, the composition according to the present invention is capable of exhibiting the effect of promoting the production of IL-10, regardless of the amount (concentration) of IL-10 in immune cells of the subject to be applied. In other words, the present composition can exhibit the effect of promoting the production of IL-10 in both a subject in a state where the concentration of IL-10 in immune cells is low and a subject in a state where the concentration of IL-10 in immune cells is high.

Since the composition according to the present invention can efficiently promote the production of IL-10, the composition can be used to ameliorate a disease such as inflammation caused by a decrease in the amount of production of IL-10.

Further, since inflammation is known to cause various kinds of diseases, the composition according to the present invention can be used not only to inhibit inflammation itself but also to ameliorate a disease caused by inflammation, and the like, by efficiently promoting the production of IL-10 known to inhibit inflammation. Therefore, the composition according to the present invention is preferably a composition for ameliorating a disease caused by inflammation. Examples of the disease caused by inflammation include metabolic syndrome, dyslipidemia, diabetes, obesity, hyperglycemia, cancers and autoimmune diseases. In the present specification, the definition of the term "ameliorating or amelioration" of a disease includes the treatment and prevention of the disease. The definition of the term "treating or treatment" of a disease includes not only stopping, alleviating or delaying the progression or worsening of the disease through medical intervention, but also stopping, alleviating or delaying the progression or worsening of the disease through non-medical intervention. The definition of the term "preventing or prevention" of a disease includes preparing for an anticipated worsening of the disease in advance and preventing the occurrence or recurrence of the disease through medical or non-medical intervention.

According to one embodiment of the present invention, the genus *Lactobacillus* lactic acid bacterium to be contained in the composition of the present invention is preferably a lactic acid bacterium having an activity to promote the production of IL-10 in immune cells of a subject who has ingested the lactic acid bacterium or a composition containing the lactic acid bacterium. The use of a lactic acid bacterium having an activity to promote the production of IL-10 in immune cells is advantageous in efficiently promoting the production of IL-10 and ameliorating inflammation, a disease caused by inflammation, and the like, in a subject who has ingested the lactic acid bacterium or a composition containing the lactic acid bacterium.

In the present specification, the expressions "promoting the production" and "having an activity to promote the production" (or "having a production promoting activity") used regarding IL-10 mean that, when bone marrow-derived dendritic cells (BMDCs) are stimulated with a composition containing a genus *Lactobacillus* lactic acid bacterium and a fermented milk, the amount of IL-10 in the stimulated BMDCs increases statistically significantly (namely, above the range of error) as compared to that in a subject who received no stimulation. The amount of IL-10 can be measured, for example, by the method described in Examples to be described later. The statistical analysis of the amount of IL-10 can be carried out by a statistical analysis method known to those skilled in the art. Specifically, the statistical analysis can be carried out by a multiple comparison test using the Tukey-Kramer method.

According to a preferred embodiment of the present invention, the composition of the present invention is a composition capable of inhibiting the production of IL-12 in the subject to be applied. Further, according to a preferred embodiment of the present invention, the composition of the present invention is a composition capable of inhibiting the production of IL-12 in immune cells of the subject to be applied, as compared to the case of administering the genus *Lactobacillus* lactic acid bacterium or the fermented milk singly. In the present specification, the expressions "the production of IL-12 of a subject", "the production of IL-12 in a subject" and the like each refer to "the production of IL-12 in immune cells of a subject".

According to a preferred embodiment of the present invention, the composition of the present invention is capable of exhibiting the effect of inhibiting the production of IL-12 regardless of the amount (concentration) of IL-12 in immune cells of the subject to be applied. In other words, according to a preferred embodiment of the present invention, the composition of the present invention can exhibit the effect of inhibiting the production of IL-12 in both a subject in a state where the amount of IL-12 in immune cells is low and a subject in a state where the amount of IL-12 in immune cells is high.

According to the descriptions in Non-patent Documents 5 and 6, since IL-12 is known to promote inflammation, it is possible to more efficiently inhibit inflammation and to more efficiently ameliorate a disease caused by inflammation, and the like, by efficiently inhibiting the production of IL-12.

According to a preferred embodiment of the present invention, the genus *Lactobacillus* lactic acid bacterium to be contained in the composition of the present invention is a lactic acid bacterium having an activity to inhibit the production of IL-12 in immune cells of a subject to whom the lactic acid bacterium is administered. The use of a lactic acid bacterium having an activity to inhibit the production of IL-12 in immune cells is advantageous in inhibiting the production of IL-12 and ameliorating inflammation, a disease caused by inflammation, and the like, in a subject to whom a composition containing the lactic acid bacterium is administered.

In the present specification, the expressions "inhibiting the production" and "having an activity to inhibit the production" (or "having a production inhibitory activity") used regarding IL-12 mean that, when BMDCs are stimulated with a composition containing a genus *Lactobacillus* lactic acid bacterium and a fermented milk, the amount of IL-12 in the stimulated BMDCs decreases statistically significantly (namely, above the range of error) as compared to that in a subject who received no stimulation. The amount of IL-12 can be measured, for example, by the method described in Examples to be described later. The statistical analysis of the amount of IL-12 can be carried out by a statistical analysis method known to those skilled in the art. Specifically, the statistical analysis can be carried out by a multiple comparison test using the Tukey-Kramer method.

According to a preferred embodiment of the present invention, the composition of the present invention is a composition capable of increasing the ratio (namely, the amount of IL-10/the amount of IL-12) of the amount of IL-10 with respect to the amount of IL-12 in BMDCs. Further, according to a preferred embodiment of the present invention, the composition of the present invention is a composition capable of increasing the above-described ratio in immune cells of the subject to be applied, as compared to the case of administering the genus *Lactobacillus* lactic acid bacterium or the fermented milk singly.

According to the descriptions in Non-patent Documents 7 to 9, since the ratio of the amount of IL-10/ the amount of IL-12 is known as an index of the inflammation inhibitory activity (anti-inflammatory activity), the composition capable of efficiently increasing the ratio of the amount of IL-10/the amount of IL-12 in immune cells of a subject can be said to be a composition having an excellent inflammation inhibitory activity.

The lactic acid bacterium to be contained in the composition according to the present invention is not particularly limited, and any genus *Lactobacillus* lactic acid bacterium can be used. The genus *Lactobacillus* lactic acid bacterium is preferably a lactic acid bacterium having an activity to promote the production of IL-10 in immune cells, and more preferably a lactic acid bacterium having both an activity to promote the production of IL-10 and an activity to inhibit the production of IL-12, in immune cells.

Examples of the lactic acid bacterium belonging to the genus *Lactobacillus* include *Lactobacillus delbrueckii subsp. burgaricus, Lactobacillus delbrueckii subsp. lactis, Lactobacillus casei, Lactobacillus helveticus, Lactobacillus acidophilus, Lactobacillus crispatus, Lactobacillus amylovorus, Lactobacillus gallinarum, Lactobacillus gasseri, Lactobacillus oris, Lactobacillus rhamnosus, Lactobacillus johnsonii, Lactobacillus fermentum, Lactobacillus brevis, Lactobacillus plantarum, Lactobacillus pentosus, Lactobacillus paraplantarum, Lactobacillus paracollinoides* and *Lactobacillus hammesii.* Among these genus *Lactobacillus* lactic acid bacteria, *Lactobacillus plantarum* is preferably used, and *Lactobacillus plantarum* strain OLL2712 is more preferably used. In the present specification, "the lactic acid bacterium belonging to the genus *Lactobacillus"* refers to a lactic acid bacterium belonging to any one of 25 genera newly established by the reorganization of lactic acid bacteria, announced in the paper "A taxonomic note on the genus Lactobacillus: Description of 23 novel genera, emended description of the genus Lactobacillus Beijerinck 1901, and union of Lactobacillaceae and Leuconostocaceae" in INTERNATIONAL JOURNAL OF SYSTEMATIC AND EVOLUTIONARY MICROBIOLOGY, Volume 70, Issue 4, published April 15, 2020. The 25 genera are, namely: the genera *Lactobacillus, Paralactobacillus, Holzapfelia, Amylolactobacillus, Bombilactobacillus, Companilactobacillus, Lapidilactobacillus, Agrilactobacillus, Schleiferilactobacillus, Loigolactobacilus, Lacticaseibacillus, Latilactobacillus, Dellaglioa, Liquorilactobacillus, Ligilactobacillus, Lactiplantibacillus, Furfurilactobacillus, Paucilactobacillus, Limosilactobacillus, Fructilactobacillus, Acetilactobacillus, Apilactobacillus, Levilactobacillus, Secundilactobacillus* and *Lentilactobacillus.* Among the lactic acid bacteria belonging to the genera after the reorganization described above, a lactic acid bacterium belonging to any of the genera *Lactobacillus, Lacticaseibacillus, Lactiplantibacillus, Liquorilactobacillus, Limosilactobacillus* and *Levilactobacillus* is preferably used in the present invention. It is noted, that conventional *Lactobacillus plantarum* is classified as the genus *Lactiplantibacillus* by the classification after the above-described reorganization, and called *Lactiplantibacillus plantarum.*

The *Lactobacillus plantarum* strain OLL2712 is deposited at the Patent Organism Depositary in the National Institute of Advanced Industrial Science and Technology (Central 6, 1-1-1 Higashi, Tsukuba, Ibaraki, Japan) on July 2, 2010, then transferred to the International Depositary under the accession number FERM BP-11262. As described in Budapest Notification No. 282 (http://www.wipo.int/treaties/en/notifications/budapest/treaty_ budapest_282.html), the National Institute of Technology and Evaluation (IPOD, NITE) has taken over the patent microorganism deposition services from the International Patent Organism Depositary of the National Institute of Advanced Industrial Science and Technology (IPOD, AIST), and therefore, the *Lactobacillus plantarum* strain OLL2712 is now deposited at the National Institute of Technology and Evaluation (IPOD, NITE) (#120, 2-5-8 Kazusakamatari, Kisarazu-shi, Chiba, Japan) under the accession number FERM BP-11262.

As the genus *Lactobacillus* lactic acid bacterium to be contained in the composition according to the present invention, a strain substantially equivalent to the above-described deposited strain can also be used. It is an unexpected fact to those skilled in the art that the above-described deposited strain or a substantially equivalent strain can promote the production of IL-10 and/or inhibit the production of IL-12. The "substantially equivalent strain" refers to, for example, a strain of the above-described genus *Lactobacillus* lactic acid bacterium, the strain having the 16S rRNA gene whose nucleotide sequence has a homology of 90% or more, preferably 95 or more, more preferably 98% or more, and still more preferably 99% or more to the nucleotide sequence (SEQ ID NO: 1) of the 16S rRNA gene of the above-described deposited strain, and preferably having the same bacteriological properties identical to those of the above-described strain. The strain having the same mycological properties is preferably a strain having an activity to promote the production of IL-10 in immune cells, at a level similar to that of the above-described deposited strain. Further, the genus *Lactobacillus* lactic acid bacterium to be contained in the composition according to the present invention may be a strain bred from the deposited strain or from a strain substantially equivalent thereto, by mutation treatment, genetic recombination, selection of a natural mutant, etc., as long as the effects of the present invention are exhibited.

Examples of the genus *Lactobacillus* lactic acid bacterium to be contained in the composition according to the present invention include, in addition to the bacterial cells themselves of the genus *Lactobacillus* lactic acid bacterium, a culture containing the bacterial cells of the genus *Lactobacillus* lactic acid bacterium. As the bacterial cells of the genus *Lactobacillus* lactic acid bacterium, both live bacterial cells and dead bacterial cells can be used. However, dead bacterial cells are preferred, and dead bacterial cells obtained by heat-treating the live bacterial cells (heat-treated dead bacterial cells) are more preferred. That is, the genus *Lactobacillus* lactic acid bacterium to be contained in the composition according to the present invention preferably includes dead bacterial cells, and more preferably includes heat-treated dead bacterial cells. The number of passages of the genus *Lactobacillus* lactic acid bacterium is not particularly limited as long as the effects of the present invention are exhibited, but is, for example, from 1 to 30, preferably from 5 to 15, and more preferably from 11 to 13.

Culturing conditions for the genus *Lactobacillus* lactic acid bacterium are not particularly limited as long as the effects of the present invention are exhibited, and conditions usually used for culturing lactic acid bacteria can be used. For example, it is possible to use, as a medium, a medium obtained by: dissolving whey powder and a whey protein concentrate in sterile water; digesting the solution with protease A; then adding thereto a yeast extract, a fish extract and MnSO₄, and further adding various nutrients (vitamins, minerals and fatty acid esters); adjusting the pH to 6.7 with NaOH, followed by autoclaving for sterilization. The pH during the culture can be adjusted within the range of from 4.8 to 6.8. K₂CO₃ can be used to adjust the pH. The temperature during the culture can be adjusted within the range of from 29 to 40°C.

The heat treatment for obtaining the heat-treated dead bacterial cells of the genus *Lactobacillus* lactic acid bacterium is not particularly limited as long as the effects of the present invention are exhibited, and is carried out under the conditions usually used for sterilizing lactic acid bacteria.

As the genus *Lactobacillus* lactic acid bacterium, it is possible to use one which has been subjected to concentration or dilution, freezing, drying, powdering, and/or the like, in addition to the heat treatment described above.

As the genus *Lactobacillus* lactic acid bacterium to be contained in the composition according to the present invention, a bacterium prepared by the above-described culture and/or any of various treatments, or a commercially available composition containing the genus *Lactobacillus* lactic acid bacterium may be used.

In the composition according to the present invention, the number of bacterial cells of the genus *Lactobacillus* lactic acid bacterium per mass of the composition is not particularly limited as long as the effects of the present invention are exhibited. However, the number of bacterial cells is preferably from 10⁶ to 10¹³ cells/g, more preferably from 10⁶ to 10¹² cells/g, still more preferably from 10⁷ to 10¹¹ cells/g, yet still more preferably from 10⁸ to 10¹¹ cells/g, and particularly preferably from 10⁸ to 10¹⁰ cells/g. Further, in the composition according to the present invention, the dry mass of bacterial cells per mass of the solid content in the composition is preferably from 0.01 to 100% by mass, more preferably from 1 to 80% by mass, and still more preferably 10 to 40% by mass.

The definition of the fermented milk to be contained in the composition according to the present invention includes "fermented milk", "dairy lactic acid bacteria beverage" and "lactic acid bacteria beverage" as defined in Ministerial Ordinance on Milk and Milk products Concerning Compositional Standards, etc. (Ministerial Ordinance on Milk and Milk products), and also includes yogurt and the like. Specifically, the fermented milk may be, for example, one obtained by fermenting milk, such as raw milk, milk, certified milk, raw goat milk, sterilized goat milk, raw sheep milk, homogenized milk, low-fat milk, non-fat mi milk or processed milk, or milk containing non-fat milk solids in an amount equal to or higher than such milk, etc., with a lactic acid bacterium or yeast, and forming the fermented product in the form of a paste or liquid, or freezing the product. These kinds of fermented milk include a set-type yogurt (solid fermented milk, set yogurt), a paste-like fermented milk (stirred yogurt) and a drink yogurt which is a liquid fermented milk. The fermented milk to be contained in the composition according to the present invention is preferably a set-type yogurt or a drink yogurt, and more preferably a drink yogurt. The average particle size of the fermented milk to be contained in the composition according to the present invention can be set as appropriate, depending on the desired properties of the composition. Specifically, the lower limit value of the average particle size of the fermented milk is preferably 0.5 µm, more preferably 1 µm, still more preferably 5 µm, yet still more preferably 10 µm and particularly preferably 15 µm, and the upper limit value thereof is preferably 100 µm, more preferably 50 µm, and still more preferably 25 µm. Further, the average particle size of the fermented milk is preferably within the range of from 0.5 to 100 µm, more preferably from 5 to 50 µm, still more preferably from 10 to 25 µm, and particularly preferably from 15 to 25 µm. The IL-10 production promoting activity can be increased to a particularly high level, by adjusting the average particle size of the fermented milk within the range described above. The average particle size of the fermented milk can be measured, for example, by a laser diffraction particle size distribution measuring apparatus (for example, SALD-2200, manufactured by Shimadzu Corporation).

The composition according to the present invention may contain a component other than the genus *Lactobacillus* lactic acid bacterium and the fermented milk, as long as the effects of the invention of the present application are not interfered with. Examples of the component other than the genus *Lactobacillus* lactic acid bacterium and the fermented milk include medium components, additives suitable for oral ingestion or tube feeding, solvents such as water, carbohydrates, proteins, lipids, vitamins, minerals, bio-essential trace metals (manganese sulfate, zinc sulfate, magnesium chloride, potassium carbonate, etc.), flavorings, carriers acceptable in terms of food hygiene or acceptable pharmaceutically, and food additives.

Examples of carbohydrates include sugars, processed starches (dextrin, soluble starches, British starches, oxidized starches, starch esters, starch ethers, etc.), and dietary fibers.

Examples of proteins include: animal and vegetable proteins such as whole milk powder, skimmed milk powder, partially skimmed milk powder, casein, whey powder, whey proteins, whey protein concentrates, whey protein isolates, α-casein, β-casein, κ-casein, β-lactoglobulin, α-lactalbumin, lactoferrin, soy proteins, egg proteins and meat proteins; hydrolyzed products of these proteins; and various milk-derived components such as butter, milk minerals, cream, whey, nonprotein nitrogen, sialic acid, phospholipids and lactose.

Examples of lipids include: animal fats and oils such as lard and fish oils, and fractionated, hydrogenated and transesterification oils thereof; and vegetable fats and oils such as palm oil, safflower oil, corn oil, rapeseed oil and coconut oil, and fractionated, hydrogenated and transesterification oils thereof.

Examples of vitamins include vitamin A, carotenes, vitamin B-complex, vitamin C, vitamin D-complex, vitamin E, vitamin K-complex, vitamin P, vitamin Q, niacin, nicotinic acid, pantothenic acid, biotin, inositol, choline and folic acid.

Examples of minerals include calcium, potassium, magnesium, sodium, copper, iron, manganese, zinc and selenium.

According to one embodiment of the present invention, the composition of the present invention for promoting the production of IL-10 can be provided as a food composition. The food composition of the present invention may be a composition for promoting an increase in the amount of IL-10, for inhibiting a decrease in the amount of IL-10, for maintaining the amount of IL-10, for inhibiting inflammation, for anti-inflammatory purposes, or for preventing and/or treating a disease such as metabolic syndrome, dyslipidemia, diabetes, obesity, hyperglycemia, a cancer or an autoimmune disease, in the subject to be applied.

The food composition of the present invention may be in any form as long as the composition is a food or beverage product capable of containing a genus *Lactobacillus* lactic acid bacterium and a fermented milk, and can be in any form that can be ingested orally or fed by tube, such as a solution, a suspension, an emulsion, a powder, a paste, a semi-solid molding or a solid molding. Specific examples of the food or beverage product include milk beverages, soft drinks, fermented milk products, lactic acid bacteria beverages, milk-based beverages, yogurts, cheeses, butters, creams, ice creams, frozen sweets, chocolates, tablets (tablet candies), gummies, breads, liquid foods, foods for the sick, nutritional foods, frozen foods, processed foods, seasonings, and other commercially available foods.

The food composition of the present invention can be produced as a food or beverage product labeled for use in promoting the production of IL-10, inhibiting inflammation, or the like. The food or beverage product can be labeled, for example, "for promoting an increase in the amount of IL-10", "for inhibiting a decrease in the amount of IL-10", "for maintaining the amount of IL-10, "for promoting the production of IL-10", "for inhibiting inflammation", "for anti-inflammatory purposes", "for preventing metabolic syndrome, dyslipidemia, diabetes, obesity, hyperglycemia, a cancer or an autoimmune disease" or the like. Labeling other than those described above can also be used equally, as long as any effect produced by promoting the production of IL-10 is indicated.

In cases where the food composition of the present invention is a composition capable of inhibiting the production of IL-12 in immune cells of the subject to be applied, the composition can be produced as a food or beverage product labeled for use in inhibiting the production of IL-12, inhibiting inflammation, or the like. The food or beverage product can be labeled, for example, "for promoting a decrease in the amount of IL-12", "for inhibiting an increase in the amount of IL-12", "for maintaining the amount of IL-12", "for inhibiting the production of IL-12", "for inhibiting inflammation", "for anti-inflammatory purposes", "for preventing metabolic syndrome, dyslipidemia, diabetes, obesity, hyperglycemia, a cancer or an autoimmune disease" or the like. Labeling other than those described above can also be used equally, as long as any effect produced by inhibiting the production of IL-12 is indicated.

In the present specification, the "labeling" means all actions to notify consumers of the above-described use, and any labeling that may allow consumers to recall or analogize the above-described use can be considered as the "labeling" in the present invention, regardless of the purpose of the labeling, the content of the labeling, the object and medium of the labeling, etc. However, it is preferred to use expressions that allow consumers to directly recognize the above-mentioned use, for the labeling.

It is preferred that the labeling is preferably one that is approved by the government, etc. (for example, approved based on various systems established by the government and labeled in a manner based on such approval). The labeling may be, for example, labeling as a health food, a functional food, a food with functional claims, an enteral food, a food for special dietary uses, a food for the sick, a food with nutrient function claims, a quasipharmaceutical product or the like. In addition, the labeling may be, for example, labeling approved by the Health, Labor and Welfare Ministry, such as a food for specified health use, or labeling approved by a similar system. Examples of the latter include labeling as a food for specified health use, labeling as a qualified food for specified health use, labeling indicating that the product may affect the structure and function of the body, and labeling indicating the effect of reducing the risk of disease. More specifically, examples include labeling as a food for specified health use (especially, labeling for health use) prescribed by Ordinance for Enforcement of Health Promotion Act (April 30, 2003, Japan Ministry of Health, Labor and Welfare Ordinance No. 86), and similar labeling.

According to one embodiment of the present invention, the composition of the present invention for promoting the production of IL-10 can be provided as a pharmaceutical composition. The pharmaceutical composition of the present invention may be a composition for promoting an increase in the amount of IL-10, for inhibiting a decrease in the amount of IL-10, for maintaining the amount of IL-10, for inhibiting inflammation, for anti-inflammatory purposes, or for preventing and/or treating a disease such as metabolic syndrome, dyslipidemia, diabetes, obesity, hyperglycemia, a cancer or an autoimmune disease, in the subject to be applied. The pharmaceutical composition of the present invention can be produced in accordance with a procedure for producing the corresponding pharmaceutical product, except for incorporating a genus *Lactobacillus* lactic acid bacterium and a fermented milk. The term "pharmaceutical composition" as used herein refers to the composition of the present invention prepared as an oral formulation or a parenteral formulation in accordance with a conventional method. At the time of formulation, additives acceptable for formulation may be used in combination. Examples of additives acceptable for formulation include excipients, stabilizers, preservatives, wetting agents, emulsifiers, lubricants, sweeteners, colorants, flavorings, buffers, antioxidants and pH adjustors. When the pharmaceutical composition is an oral formulation, the composition can be prepared as a solid formulation such as a tablet, a powder, a fine granule, a granule, a capsule or a sustained-release agent, or a liquid formulation such as a solution, a suspension or an emulsion. When the pharmaceutical composition is a parenteral formulation, the composition can be prepared as an injection, a suppository or the like. The pharmaceutical composition is preferably an oral formulation from the viewpoint of ease of ingestion by (or administration to) a target subject.

One embodiment of the present invention provides an anti-inflammatory composition containing a genus *Lactobacillus* lactic acid bacterium and a fermented milk.

The amount of application (the amount of ingestion or the amount of administration) of the composition according to the present invention is not particularly limited as long as the effects of the present invention are exhibited, and can be adjusted as appropriate depending on the age, health status, body weight and the like of the subject to be applied. Typically, the amount of application of the composition is from 0.001 to 10 g/kg body weight, preferably from 0.01 to 7 g/kg body weight, more preferably from 0.5 to 5 g/kg body weight, and still more preferably from 1 to 2 g/kg body weight, per day. The dry mass of bacterial cells of the genus *Lactobacillus* lactic acid bacterium is preferably from 0.001 to 1,000 mg/kg body weight, more preferably from 0.01 to 100 mg/kg body weight, still more preferably from 0.05 to 30 mg/kg body weight, and yet still more preferably 0.1 to 10 mg/kg body weight. The number of bacterial cells of the genus *Lactobacillus* lactic acid bacterium is preferably from 10⁴ to 10¹² cells/kg body weight, more preferably from 10⁵ to 10¹¹ cells/kg body weight, still more preferably from 10⁶ to 10¹⁰ cells/kg body weight, and particularly preferably from 10⁶ to 10⁸ cells/kg body weight.

The composition according to the present invention is preferably applied (ingested or administered) continuously over a long period of time, in order to better exert its effect. Specifically, the composition is preferably applied continuously for 3 days or more, and more preferably applied continuously for 1 week or more. The period of application may be, for example, from 1 to 6 weeks, from 1 to 12 weeks, from 2 to 10 weeks, from 4 to 10 weeks or from 4 to 12 weeks. In the present specification, the term "continuously" means to continue applying a predetermined amount of the composition according to the present invention every day.

### Method of Producing Composition for Promoting Production of IL-10

The composition according to the present invention can be produced by incorporating a genus *Lactobacillus* lactic acid bacterium and a fermented milk, and in addition, a pharmaceutically acceptable carrier and/or additives, a carrier acceptable in terms of food hygiene and/or additives, etc., as described above. Therefore, another aspect of the present invention provides a method of producing a composition for promoting the production of IL-10, the method including the step of incorporating a genus *Lactobacillus* lactic acid bacterium and a fermented milk.

One aspect of the present invention provides a method of producing a composition for promoting the production of IL-10, the method including the following steps:
a starter inoculation step of inoculating a raw material milk with a starter to obtain a fermented milk base material;
a fermentation step of fermenting the fermented milk base material to obtain a fermented milk;
a lactic acid bacterium addition step of adding a lactic acid bacterium belonging to the genus *Lactobacillus* to the fermented milk, to obtain a lactic acid bacterium-added fermented milk; and
a stirring step of stirring at least one selected from the group consisting of the fermented milk and the lactic acid bacterium-added fermented milk.

In the production method according to the present invention, any known raw material milk can be used depending on the type of the desired composition. The raw material milk contains one or more selected from the group consisting of milk, condensed milk, whole milk powder, skimmed milk, condensed skimmed milk, skimmed milk powder, partially skimmed milk, condensed partially skimmed milk, partially skimmed milk powder and a milk protein concentrate. For example, raw milk can be used as the raw material milk. Further, it is also possible to use a mixture obtained by adding, to the raw milk, sterilized milk, whole milk, skimmed milk, condensed whole milk, condensed skimmed milk, whole milk powder, skimmed milk powder, butter milk, salted butter, unsalted butter, whey, whey powder, a whey protein concentrate (WPC), a whey protein isolate (WPI), o-lactalbumin (o-La), β-lactoglobulin (β-Lg), lactose, cream, water and/or the like.

The raw material milk may be one that has been subjected to a homogenization treatment in advance. In the homogenization treatment, particles (fat globules) mainly composed of proteins and/or fats contained in the raw material milk are finely crushed (refined). The homogenization treatment may be carried out only once, or two or more times. The raw material milk can be homogenized, for example, by: a method of passing the raw material milk through a narrow gap while pushing the milk with a pressure; a method of passing the raw material milk through a narrow gap while suctioning the milk under reduced pressure; or the like.

The raw material milk may be one that has been subjected to a sterilization treatment in advance. The sterilization treatment is carried out by heating the raw material milk while adjusting the heating temperature and the heating time to such degrees that unwanted bacteria contained in the raw material milk can be sterilized. The heating temperature of the raw material milk is, for example, 80°C or higher, or 90°C or higher. The sterilization treatment can be carried out, for example, by a known method such as high-temperature short-time sterilization (HTST), ultra-high temperature sterilization (UHT) or the like. When the raw material milk is sterilized by heating, the raw material milk heated to a high temperature is preferably cooled to a temperature range (fermentation temperature range) suitable for fermentation, before the starter inoculation step. The fermentation temperature refers to a temperature at which a microorganism such as a lactic acid bacterium is activated to promote the growth of the microorganism. For example, the fermentation temperature range of the raw material milk is from 30 to 60°C.

The respective steps in the production method according to the present invention will be described below.

### < Starter Inoculation Step >

The starter inoculation step is a step of inoculating a raw material milk with a starter (namely, a step of adding a starter to a raw material milk). The amount of the starter with which the raw material milk is inoculated is, for example, 0.01% by mass or more, from 0.01 to 15% by mass, from 0.05 to 10% by mass or from 0.11 to 5% by mass, with respect to the total mass of the raw material milk. In the present specification, the raw material milk inoculated with the starter is also referred to as "fermented milk base material".

The starter contains one or both of a lactic acid bacterium and a yeast. The starter preferably contains, as the lactic acid bacterium, *Lactobacillus delbrueckii,* and more preferably *Lactobacillus delbrueckii subsp. burgaricus.* The starter still more preferably contains *Streptococcus thermophilus* in addition to *Lactobacillus delbrueckii subsp. Burgaricus.* Further, the starter may contain a known lactic acid bacterium and/or *Bifidobacterium,* in addition to, or instead of, the above-described *Lactobacillus delbrueckii subsp. Burgaricus* and *Streptococcus thermophilus.* Examples of the known lactic acid bacterium include *Lactobacillus gasseri, Lactobacillus plantarum, Lactobacillus casei, Lactococcus lactis* and *Lactococcus lactis subsp. cremoris.* Examples of the known *Bifidobacterium* include *Bifidobacterium longum* and *Bifidobacterium bifidum.* The lactic acid bacterium to be contained in the starter may be the same as, or different from the lactic acid bacterium to be added to the fermented milk in the lactic acid bacterium addition step to be described later. However, a different lactic acid bacterium is preferably used.

### < Fermentation Step >

The fermentation step is a step of fermenting the raw material milk with the starter. In the fermentation step, the raw material milk (fermented milk base material) inoculated with the starter is maintained in the fermentation temperature range (for example, from 30 to 60°C) to ferment the raw material milk, to obtain a fermented milk. The fermentation temperature and the fermentation time as fermentation conditions are adjusted as appropriate, depending on the type of the raw material milk, the type and the amount of the starter, the desired flavor and texture of the fermented milk, and the like. Specifically, the raw material milk is maintained in the fermentation temperature range for 1 hour or more, preferably from 1 to 12 hours, more preferably from 2 to 8 hours, and still more preferably from 3 to 5 hours. Further, the fermentation conditions of the raw material milk may be set as the lactic acid acidity (acidity) and the pH of the resulting fermented milk. For example, in cases where solidsnon-fat (SNF) of the raw material milk is from 8 to 10% by mass, the fermentation condition is set such that the fermentation is continued until the lactic acid acidity of the resulting fermented milk reaches, for example, 0.7% or more, or 0.8% or more. The acidity of the raw material milk is measured in accordance with "Test Method for Compositional Standards for Milk and Milk Products" under the Ministerial Ordinance on Milk and Milk products.

### < Lactic Acid Bacterium Addition Step >

The lactic acid bacterium addition step is a step of adding a genus *Lactobacillus* lactic acid bacterium to at least one selected from the group consisting of the raw material milk, the fermented milk base material and the fermented milk, to obtain a lactic acid bacterium-containing composition. The genus *Lactobacillus* lactic acid bacterium is added to the raw material milk before the addition of the starter, the fermented milk base material after the addition of the starter, the fermented milk during the fermentation of the fermented milk base material, and/or the fermented milk after the fermentation of the fermented milk base material. The same genus *Lactobacillus* lactic acid bacterium as one contained in the composition for promoting the production of interleukin-10 can be used, as the genus *Lactobacillus* lactic acid bacterium. The genus *Lactobacillus* lactic acid bacterium is preferably *Lactobacillus plantarum,* and more preferably *Lactobacillus plantarum* strain OLL2712. It is possible to use a culture liquid, a concentrated culture liquid, frozen concentrated bacterial cells, frozen pellets, a freeze-dried powder or the like, as the genus *Lactobacillus* lactic acid bacterium. In cases where the starter to be inoculated to the raw material milk in the above-described starter inoculation step contains a genus *Lactobacillus* lactic acid bacterium, the genus *Lactobacillus* lactic acid bacterium contained in the starter may be the same as, or different from, the lactic acid bacterium to be added in the present lactic acid bacterium addition step. According to a preferred embodiment of the present invention, the genus *Lactobacillus* lactic acid bacterium contained in the starter used in the starter inoculation step is different from the lactic acid bacterium to be added in the lactic acid bacterium addition step.

The amount of the genus *Lactobacillus* lactic acid bacterium to be added to the raw material milk, the fermented milk base material and/or the fermented milk is, for example, 0.01% by mass or more, from 0.01 to 15% by mass, from 0.02 to 10% by mass or from 0.03 to 5% by mass, and particularly preferably from 0.03 to 1% by mass, with respect to the total mass of the raw material milk, the fermented milk base material or the fermented milk to which the genus *Lactobacillus* lactic acid bacterium is to be added. The number of bacterial cells of the genus *Lactobacillus* lactic acid bacterium to be added to the fermented milk is preferably from 10⁶ cells/g to 10¹² cells/g, more preferably from 10⁷ cells/g to 10¹¹ cells/g, and still more preferably from 10⁸ cfu/g to 10¹⁰ cells/g.

### < Stirring Step>

The stirring step is a step of stirring the raw material milk, the fermented milk base material, the fermented milk and/or the lactic acid bacterium-containing composition. The IL-10 production promoting activity in the composition obtained by the production method according to the present invention can be increased to a particularly high level, by stirring the lactic acid bacterium-containing composition.

Stirring conditions in the stirring step can be set as appropriate depending on the size, the shape and the like of the container, stirring device, etc., to be used in the stirring, the stirring speed, the stirring time and the like. In cases where the object to be stirred is the fermented milk containing the genus *Lactobacillus* lactic acid bacterium, for example, conditions are set as appropriate such that the lower limit value of the average particle size of the fermented milk after stirring is preferably 0.5 µm, more preferably 5 µm, still more preferably 10 µm, and particularly preferably 15 µm, and the upper limit value thereof is preferably 100 µm, more preferably 50 µm, and still more preferably 25 µm. The average particle size of the fermented milk after stirring can be adjusted preferably within the range of from 0.5 to 100 µm, more preferably from 1 to 50 µm, still more preferably from 5 to 50 µm, yet still more preferably from 10 to 25 µm, and particularly preferably from 15 to 25 µm. The IL-10 production promoting activity can be increased to a particularly high level, by adjusting the average particle size of the fermented milk containing the genus *Lactobacillus* lactic acid bacterium within the range described above. In the present invention, the average particle size of the fermented milk containing the genus *Lactobacillus* lactic acid bacterium can be measured by the same method as that used for the measurement of the average particle size of the fermented milk in the composition according to the present invention described above. For example, in the case of stirring 1,000 g of the raw material milk, the fermented milk base material, the fermented milk or the lactic acid bacterium-containing composition, using a stainless steel tray (diameter: 15 cm) with a capacity of 3.0 L and a T-shaped stirring blade with a diameter of 10 cm, the lower limit value of the stirring speed can be adjusted to 30 rpm, 50 rpm, 100 rpm, 150 rpm, 160 rpm or 200 rpm, and the upper limit value of the stirring speed can be adjusted to 500 rpm, 400 rpm, 350 rpm, 300 rpm or 250 rpm. The stirring speed can be adjusted preferably within the range of from 30 to 500 rpm, more preferably from 50 to 400 rpm, and still more preferably from 100 to 200 rpm. The stirring may be carried out continuously or intermittently, but is preferably carried out continuously from the viewpoints of removing the carbon dioxide gas generated and preventing solids from floating up. It is also possible to use a known paddle-shaped stirring blade, mixer or food cutter in the stirring step. Further, the shear force at which the lactic acid bacterium-containing composition is sheared during the stirring step can be adjusted as appropriate depending on the type of the stirrer (shearing machine) to be used, the stirring conditions and the like.

### < Cooling Step >

The production method according to the present invention may further include a cooling step of cooling the lactic acid bacterium-containing composition.

In the cooling step, the lactic acid bacterium-containing composition is cooled until a temperature lower than the fermentation temperature range (for example, from 30 to 60°C), for example, 15°C or lower, is reached. The lactic acid bacterium-containing composition is cooled preferably to a temperature of from 1 to 15°C, more preferably from 3 to 12°C, and still more preferably from 5 to 10°C. The cooling method (cooling conditions) is not particularly limited, and the composition may be cooled rapidly by ice cooling or the like, or may be cooled slowly in a thermostatic device such as a refrigerator, or in a thermostatic chamber, etc. The expression "to cool rapidly" refers to, for example, a cooling method in which the lactic acid bacterium-containing composition within the fermentation temperature range, at around 40°C, is cooled to 10°C or lower over a time period of from about 10 to about 30 minutes. On the other hand, the expression "to cool slowly" refers to, for example, a cooling method in which the lactic acid bacterium-containing composition within the fermentation temperature range, at around 40°C, is cooled to 10°C or lower over a time period of from about 1 to about 3 hours.

The cooling step may be carried out singly, or may be carried out concurrently with the stirring step described above. The cooling step is preferably carried out concurrently with stirring step, from the viewpoints of enhancing the production efficiency of the composition, and enhancing the IL-10 production promoting activity in the resulting composition. In the case of carrying out the stirring step and the cooling step concurrently, the same conditions as those described in the stirring step and the cooling step can be used, as the stirring conditions and the cooling conditions, respectively.

One aspect of the present invention provides a method of promoting the production of IL-10 in a subject, the method including applying an effective amount of a combination of a genus *Lactobacillus* lactic acid bacterium and a fermented milk to the subject. Further, according to a preferred embodiment of the present invention, the above-described method is a method for ameliorating inflammation in a subject. The method for ameliorating inflammation in a subject may be a non-therapeutic method, or may be a therapeutic method. According to one embodiment of the present invention, the therapeutic method is provided as a method of ameliorating a disease caused by inflammation, or the like. According to another preferred embodiment, the amelioration in the therapeutic method is treatment and/or prevention. Therefore, one embodiment is a method of treating and/or preventing inflammation in a subject, the method including applying an effective amount of a combination of a genus *Lactobacillus* lactic acid bacterium and a fermented milk to the subject. Another embodiment is a method of treating and/or preventing a disease caused by inflammation in a subject, the method including applying an effective amount of a combination of a genus *Lactobacillus* lactic acid bacterium and a fermented milk to the subject.

Examples of the disease caused by inflammation include, but not particularly limited to, metabolic syndrome, dyslipidemia, diabetes, obesity, hyperglycemia, cancers and autoimmune diseases. Therefore, still another embodiment is a method of treating and/or preventing a disease selected from the group consisting of metabolic syndrome, dyslipidemia, diabetes, obesity, hyperglycemia, cancers and autoimmune diseases, in a subject, the method including applying an effective amount of a combination of a genus *Lactobacillus* lactic acid bacterium and a fermented milk to the subject.

Further, the subject to be applied of the combination of a genus *Lactobacillus* lactic acid bacterium and a fermented milk is not particularly limited, as long as the subject is in need of promoting the production of IL-10. Further, the subject to be applied may be a healthy individual, or alternatively, may be an individual with a decreased IL-10 production or an individual in whom the production of IL-10 has a tendency to decrease.

In the method of promoting the production of IL-10, the amount and period of application of the combination of a₋₋genus *Lactobacillus* lactic acid bacterium and a fermented milk are not particularly limited as long as the effects of the present invention are exhibited, and can be adjusted as appropriate depending on the age, health status, body weight and the like of the subject to be applied. Typically, the amount and period of application of the combination of a genus *Lactobacillus* lactic acid bacterium and a fermented milk are the same as the amount and period of application of the composition according to the present invention.

Another aspect of the present invention provides the use of a composition containing a genus *Lactobacillus* lactic acid bacterium and a fermented milk for promoting the production of IL-10. According to one embodiment of the present invention, the above-described use is provided as the use of a composition containing a genus *Lactobacillus* lactic acid bacterium and a fermented milk, for treating and/or preventing inflammation. According to still another embodiment, the above-described use is provided as the use of a composition containing a genus *Lactobacillus* lactic acid bacterium and a fermented milk, for treating and/or preventing a disease caused by inflammation.

Examples of the disease caused by inflammation include, but not particularly limited to, metabolic syndrome, dyslipidemia, diabetes, obesity, hyperglycemia, cancers and autoimmune diseases. Therefore, still another embodiment is the use of a composition containing a genus *Lactobacillus* lactic acid bacterium and a fermented milk, for treating and/or preventing a disease selected from the group consisting of metabolic syndrome, dyslipidemia, diabetes, obesity, hyperglycemia, cancers and autoimmune diseases.

According to a preferred embodiment, the composition containing a genus *Lactobacillus* lactic acid bacterium and a fermented milk is provided as a food composition. According to another preferred embodiment, the composition containing a genus *Lactobacillus* lactic acid bacterium and a fermented milk is applied to a subject by a non-therapeutic method.

Another aspect of the present invention provides the use of a genus *Lactobacillus* lactic acid bacterium and a fermented milk for the production of a composition for promoting the production of IL-10. According to one embodiment of the present invention, the above-described use is provided as the use of a genus *Lactobacillus* lactic acid bacterium and a fermented milk, for the production of a composition for treating and/or preventing inflammation. According to still another embodiment, the above-described use is provided as the use of a composition containing a genus *Lactobacillus* lactic acid bacterium and a fermented milk, for the production of a composition for treating and/or preventing a disease caused by inflammation.

Examples of the disease caused by inflammation include, but not particularly limited to, metabolic syndrome, dyslipidemia, diabetes, obesity, hyperglycemia, cancers and autoimmune diseases. Therefore, still another embodiment is the use of a composition containing a genus *Lactobacillus* lactic acid bacterium and a fermented milk, for the production of a composition for treating and/or preventing a disease selected from the group consisting of metabolic syndrome, dyslipidemia, diabetes, obesity, hyperglycemia, cancers and autoimmune diseases.

According to a preferred embodiment, the genus *Lactobacillus* lactic acid bacterium and the fermented milk are used for the production of a food composition. According to another preferred embodiment, the genus *Lactobacillus* lactic acid bacterium and the fermented milk are applied to a subject by a non-therapeutic method.

Another aspect of the present invention provides the use of a composition containing a genus *Lactobacillus* lactic acid bacterium and a fermented milk for the production of a pharmaceutical product for treating a disease caused by a decrease in the amount of production of IL-10. According to a preferred embodiment, the disease caused by a decrease in the amount of production of IL-10 is inflammation, a disease caused by inflammation, or the like. Therefore, one embodiment is the use of a composition containing a genus *Lactobacillus* lactic acid bacterium and a fermented milk, for the production of a pharmaceutical product for treating inflammation. Another embodiment is the use of a composition containing a genus *Lactobacillus* lactic acid bacterium and a fermented milk, for the production of a pharmaceutical product for treating a disease caused by inflammation.

Examples of the disease caused by inflammation include, but not particularly limited to, metabolic syndrome, dyslipidemia, diabetes, obesity, hyperglycemia, cancers and autoimmune diseases. Therefore, still another embodiment is the use of a composition containing a genus *Lactobacillus* lactic acid bacterium and a fermented milk, for the production of a pharmaceutical product for treating a disease selected from the group consisting of metabolic syndrome, dyslipidemia, diabetes, obesity, hyperglycemia, cancers and autoimmune diseases.

Another aspect of the present invention provides a method of promoting the production of IL-10 in a subject in need thereof, the method including applying an effective amount of a composition containing a genus *Lactobacillus* lactic acid bacterium and a fermented milk to the subject. A preferred embodiment of the present invention provides a method of inhibiting inflammation or a disease caused by inflammation in a subject in need thereof, the method including applying an effective amount of a composition containing a genus *Lactobacillus* lactic acid bacterium and a fermented milk to the subject. The definition of the term "inhibiting or inhibition" includes not only a concept of treating a pathological condition that has been established, but also of preventing a pathological condition which may possibly be established in the future. Further, the definition of the term "inhibiting or inhibition" also includes a non-therapeutic concept, for example, a concept of ameliorating or alleviating inflammation, a disease caused by inflammation, and the like. Therefore, one embodiment is a method of ameliorating and/or alleviating inflammation in a subject in need thereof, the method including applying an effective amount of a composition containing a genus *Lactobacillus* lactic acid bacterium and a fermented milk to the subject. Another embodiment is a method of ameliorating and/or alleviating a disease caused by inflammation in a subject in need thereof, the method including applying an effective amount of a composition containing a genus *Lactobacillus* lactic acid bacterium and a fermented milk to the subject.

Another aspect of the present invention provides the use of a composition containing a genus *Lactobacillus* lactic acid bacterium and a fermented milk for the production of a food or beverage product for treating a disease caused by a decrease in the amount of production of IL-10. According to a preferred embodiment, the disease caused by a decrease in the amount of production of IL-10 is inflammation, a disease caused by inflammation, or the like. Therefore, one embodiment is the use of a composition containing a genus *Lactobacillus* lactic acid bacterium and a fermented milk, for the production of a food or beverage product for treating inflammation. Another embodiment is the use of a composition containing a genus *Lactobacillus* lactic acid bacterium and a fermented milk, for the production of a food or beverage product for treating a disease caused by inflammation.

Examples of the disease caused by inflammation include, but not particularly limited to, metabolic syndrome, dyslipidemia, diabetes, obesity, hyperglycemia, cancers and autoimmune diseases. Therefore, still another embodiment is the use of a composition containing a genus *Lactobacillus* lactic acid bacterium and a fermented milk, for the production of a food or beverage product for treating a disease selected from the group consisting of metabolic syndrome, dyslipidemia, diabetes, obesity, hyperglycemia, cancers and autoimmune diseases.

Examples of the disease caused by inflammation include, but not particularly limited to, metabolic syndrome, dyslipidemia, diabetes, obesity, hyperglycemia, cancers and autoimmune diseases. Therefore, still another embodiment is a method of ameliorating and/or alleviating a disease selected from the group consisting of metabolic syndrome, dyslipidemia, diabetes, obesity, hyperglycemia, cancers and autoimmune diseases in a subject in need thereof, the method including applying an effective amount of a composition containing a genus *Lactobacillus* lactic acid bacterium and a fermented milk to the subject.

### EXAMPLES

The present invention will be specifically described with reference to the following examples, but the present invention is not limited to these examples. It is noted that units and measurement methods used in the present Examples are in accordance with JIS (Japanese Industrial Standards) unless otherwise defined. Further, the statistical analysis of the amount of IL-10 and the amount of IL-12 in the present Examples was carried out by a multiple comparison test using the Tukey-Kramer method, unless otherwise defined.

### Example 1: Examination of Method of Producing Composition Containing Genus Lactobacillus Lactic Acid Bacterium and Fermented milk

The examination of the method of producing a composition containing a genus *Lactobacillus* lactic acid bacterium and a fermented milk was carried out, from the viewpoint of the IL-10 production promoting activity of the resulting composition.

### (1) Fluctuation in IL-10 Production Promoting Activity in Production Process of Composition Containing genus Lactobacillus lactic acid bacterium and Fermented milk

A lactic acid bacteria starter A composed of *Lactobacillus delbrueckii subsp. burgaricus* and *Streptococcus thermophilus,* and the lactic acid bacterium *Lactobacillus plantarum* strain OLL2712 (Accession No: FERM BP-11262) which had been subjected to a heat treatment were added to a raw material milk (a mixture containing raw milk, skimmed milk powder, cream, sugar and stevia), and the resulting mixture was maintained at 43°C until a pH of from about 4.4 about to 4.6 was reached (from 4 to 6 hours) to ferment the raw material milk, to prepare a fermented milk. The heat treatment of the lactic acid bacterium *Lactobacillus plantarum* strain OLL2712 was carried out by concentrating the bacterial cells and then heating the bacterial cells at 60°C for 10 minutes. Subsequently, the fermented milk was cooled slowly to 4°C in a refrigerator, while stirring at 150 rpm. Thereafter, the fermented milk was homogenized by a homomixer, to obtain a drink yogurt. The amount of bacterial cells of the lactic acid bacterium *Lactobacillus plantarum* strain OLL2712 contained in the fermented milk was 5 × 10⁹ or more cells/112 g.

During the process until the fermented milk was homogenized, the raw material milk at the start of the fermentation (pH: about 6.5), the fermented milk 3 hours after the start of the fermentation (pH: from about 4.8 to about 5.0), the fermented milk before the stirring and cooling (pH: from about 4.4 to about 4.6), the fermented milk after the stirring and cooling (pH: from about 4.2 to about 4.4), and the fermented milk after the homogenization, were collected respectively, and the samples were stored at -80°C.

The raw material milk sample and the respective fermented milk samples stored at -80°C were each thawed, and the IL-10 production promoting activity in bone marrow-derived dendritic cells (BMDCs) was measured for each sample as follows.

Bone marrow fluid extracted from the femur of BALB/c mice (produced by Japan SLC, Inc.) was passed through a 70 µm cell strainer and hemolyzed, and then rabbit IgG was added to prevent non-specific binding of antibodies. Subsequently, biotinlabeled anti-CD4 antibody, anti-CD8 antibody and I-Ad (MHCII marker) antibody were added, and the resulting mixture was left to stand on ice for 30 minutes. To the resultant, streptavidin magnetic beads and anti-B220 antibody magnetic beads were added. After passing through a 40 µm cell strainer, the negative fraction was collected by auto MACS DEPLETE. By this operation, T cells, B cells and antigen-presenting cells were removed from the cells in the bone marrow fluid, and only immature dendritic cells could be separated.

The obtained immature dendritic cells were cultured in 10 ml of RPMI-10 containing 10% GM-CSF, and 5 ml of the RPMI-10 containing GM-CSF was added to the culture 3 days later. After another 5 days, the floating cells were collected, and used as BMDCs.

The obtained BMDCs were seeded in a 96-well plate at a concentration of 10⁵ cells/well. Subsequently, the raw material milk sample or each fermented milk sample was added to each well at a concentration of from 0.5 to 2%, followed by culturing. After 24 hours, the culture supernatant was collected, and the amount (concentration) of IL-10 in the collected supernatant was measured using a mouse ELISA kit to determine the amount of production of IL-10 in the BMDCs. Both the antibodies and ELISA kit used were purchased from Becton, Dickinson and Company. The results of the amount of production of IL-10 in BMDCs measured for each of the raw material milk sample and the respective fermented milk samples (namely, the IL-10 production promoting activity of each sample) are shown in FIG. 1.

The results in FIG. 1 have shown that the production of IL-10 is significantly promoted in cases where the fermented milk sample collected at each time point before the stirring and cooling, after the stirring and cooling or after the homogenization, is added, as compared to the case where the raw material milk sample collected before the start of the fermentation is added. Particularly in cases where the fermented milk sample collected at each time point after the stirring and cooling or after the homogenization is added, it has been shown that the production of IL-10 is especially markedly promoted. These results suggest that the IL-10 production promoting activity is increased by a composition containing a genus *Lactobacillus* lactic acid bacterium and a fermented milk, and further, that the IL-10 production promoting activity of the composition containing a genus *Lactobacillus* lactic acid bacterium and a fermented milk is especially markedly increased by stirring and cooling the composition.

### (2) Examination of Conditions for Stirring and Cooling Steps: No. 1

The conditions for the stirring and cooling steps in the production of a composition containing a genus *Lactobacillus* lactic acid bacterium and a fermented milk (drink yogurt) were examined in accordance with the following procedure.

The lactic acid bacteria starter A and the lactic acid bacterium *Lactobacillus plantarum* strain OLL2712 which had been subjected to a heat treatment were added to a raw material milk (a mixture containing raw milk, skimmed milk powder, cream, sugar and stevia), and the resulting mixture was maintained at 43°C until a pH of from about 4.4 to about 4.6 was reached (from 4 to 6 hours) to ferment the raw material milk, to prepare a fermented milk. The heat treatment of the lactic acid bacterium *Lactobacillus plantarum* strain OLL2712 was carried out by concentrating the bacterial cells and then heating the bacterial cells at 60°C for 10 minutes. The amount of bacterial cells of the lactic acid bacterium *Lactobacillus plantarum* strain OLL2712 contained in the resulting fermented milk was 5 × 10⁹ or more cells/112 g. The thus obtained fermented milk was divided into 3 groups, and the fermented milk of each group was cooled under the following conditions.
Group 1: stirred while cooling rapidly with ice.
Group 2: stirred while cooling slowly in a refrigerator.
Group 3: left to stand in a refrigerator to cool slowly without stirring.

The stirring in Group 1 and Group 2 was carried out at 150 rpm. In the rapid cooling in Group 1, a time period of about 20 minutes was required until the temperature of the lactic acid bacterium-added fermented milk reached 10°C or lower. On the other hand, in the slow cooling in Group 2 and Group 3, a time period of about 2 hours was required until the temperature of the lactic acid bacterium-added fermented milk reached 10°C or lower.

The fermented milk of each group after the stirring and cooling was collected, and the samples were stored at -80°C. Each fermented milk sample after the storage was thawed, and the IL-10 production promoting activity in BMDCs was measured for each fermented milk sample, in the same manner as in the section (1) described above. The results are shown in FIG. 2.

The results in FIG. 2 have shown that the production of IL-10 is significantly promoted in both Group 1 in which stirring was performed while cooling rapidly and Group 2 in which stirring was performed while cooling slowly, as compared to Group 3 in which cooling was performed slowly without stirring. The above-described results suggest that the IL-10 production promoting activity of a composition containing a genus *Lactobacillus* lactic acid bacterium and a fermented milk is markedly increased by performing stirring in the production process of the composition.

### (3) Examination of Conditions for Stirring and Cooling Steps: No. 2

The conditions for the stirring and cooling steps in the production of a composition containing a genus *Lactobacillus* lactic acid bacterium and a fermented milk were examined in accordance with the following procedure.

The lactic acid bacteria starter A and the lactic acid bacterium *Lactobacillus plantarum* strain OLL2712 which had been subjected to a heat treatment were added to a raw material milk (a mixture containing raw milk, skimmed milk powder, cream, sugar and stevia), and the resulting mixture was maintained at 43°C until a pH of from about 4.4 to about 4.6 was reached (from 4 to 6 hours) to ferment the raw material milk, to prepare a lactic acid bacterium-containing fermented milk. The heat treatment of the lactic acid bacterium *Lactobacillus plantarum* strain OLL2712 was carried out by concentrating the bacterial cells and then heating the bacterial cells at 60°C for 10 minutes. The amount of bacterial cells of the lactic acid bacterium *Lactobacillus plantarum* strain OLL2712 contained in the resulting lactic acid bacterium-containing fermented milk was 5 × 10⁹ or more cells/112 g. Meanwhile, a control fermented milk was prepared in the same manner as the lactic acid bacterium-containing fermented milk described above, except that the lactic acid bacterium *Lactobacillus plantarum* strain OLL2712 was not added. Each of the lactic acid bacterium-containing fermented milk and the control fermented milk was divided into two groups, and the fermented milk of each group was cooled and stirred under the following conditions.
Group 1 (control fermented milk): left to stand in a refrigerator to cool slowly without stirring.
Group 2 (control fermented milk): stirred while cooling slowly in a refrigerator.
Group 3 (lactic acid bacterium-containing fermented milk): left to stand in a refrigerator to cool slowly without stirring.
Group 4 (lactic acid bacterium-containing fermented milk): stirred while cooling slowly in a refrigerator.

The fermented milk of each group was collected before and after the cooling, and the samples were stored at -80°C. Each fermented milk sample after the storage was thawed, and the IL-10 production promoting activity in BMDCs was measured for each fermented milk sample, in the same manner as in the section (1) described above. The results are shown in FIG. 3.

The results in FIG. 3 have shown that, in the lactic acid bacterium-containing fermented milk, the production of IL-10 is significantly promoted in both cases where static cooling was performed (Group 3) and stirring and cooling was performed (Group 4), as compared to the control fermented milk. Further, it has been shown that the production of IL-10 is significantly promoted in the lactic acid bacterium-containing fermented milk (Group 4) in which stirring and cooling was performed, as compared to the lactic acid bacterium-containing fermented milk (Group 3) in which static cooling was performed. These results suggest that the IL-10 production promoting activity is increased by a composition containing a genus *Lactobacillus* lactic acid bacterium and a fermented milk, and further, that the IL-10 production promoting activity of the composition containing a genus *Lactobacillus* lactic acid bacterium is increased by performing stirring in the production process of the composition, thereby leading to a particularly marked increase in the IL-10 production promoting activity of the composition.

### Example 2: Confirmation of Effects of Composition Containing Genus Lactobacillus Lactic Acid Bacterium and Fermented milk on Cytokines

The effects of a composition containing a genus *Lactobacillus* lactic acid bacterium and a fermented milk on the production of an anti-inflammatory cytokine (IL-10) and a proinflammatory cytokine (IL-12) were examined.

### (1) Effects on Cytokine Production Promoting Activity: No. 1

A lactic acid bacteria starter B which is different from the lactic acid bacteria starter A and composed of *Lactobacillus delbrueckii subsp. burgaricus* and *Streptococcus thermophilus,* and the lactic acid bacterium *Lactobacillus plantarum* strain OLL2712 which had been subjected to a heat treatment, were added to a raw material milk (a mixture containing raw milk, skimmed milk powder, cream, sugar and stevia). The resulting mixture was maintained at 43°C until a lactic acid acidity (acidity) of 0.7% was reached to ferment the raw material milk, to prepare a lactic acid bacterium-containing fermented milk (set yogurt). The heat treatment of the lactic acid bacterium was carried out by concentrating the bacterial cells and then heating the bacterial cells at 60°C for 10 minutes. The amount of bacterial cells of the genus *Lactobacillus* lactic acid bacterium contained in the resulting lactic acid bacterium-containing fermented milk was 5 × 10⁹ or more cells/112 g. Meanwhile, a control fermented milk (set yogurt) was prepared in the same manner as the lactic acid bacterium-containing fermented milk described above, except that the genus *Lactobacillus* lactic acid bacterium *Lactobacillus plantarum* strain OLL2712 was not added.

The amount of production of the anti-inflammatory cytokine IL-10 was measured in the same manner as in the above-described Example 1, except that each fermented milk sample one day after the preparation was added to bone marrow-derived dendritic cells (10⁵ cells/well) at a concentration of 1%. Further, the amount of production of IL-10 was measured in the same manner as the measurement of the amount of production of IL-10 in the above-described Example 1, except that "Mouse IL-12 (p70) ELISA Set" (manufactured by Becton Dickinson Company) was used as the ELISA kit. The amount of production (concentration) of IL-12 as used herein was measured as the amount of production (concentration) of active IL-12 (p70). The amounts of production of IL-10 and IL-12 in bone marrow-derived dendritic cells were measured in the same manner as described above, except that the lactic acid bacterium *Lactobacillus plantarum* strain OLL2712 alone was added such that the amount of bacterial cells was about the same as those contained in the lactic acid bacterium-containing fermented milk. The measured results of the amount of production of IL-10 and the amount of production of IL-12 are shown in FIG. 4A and FIG. 4B, respectively.

The results in FIG. 4A have shown that the production of IL-10 is significantly promoted in cases where the lactic acid bacterium-containing fermented milk containing the genus *Lactobacillus* lactic acid bacterium and the fermented milk is added, as compared to the case where the genus *Lactobacillus* lactic acid bacterium or the fermented milk (control fermented milk) is added singly. Further, it has been shown that the amount of production of IL-10 when the lactic acid bacterium-containing fermented milk containing the genus *Lactobacillus* lactic acid bacterium and the fermented milk is added, is higher than the total of the respective amounts of production of IL-10 when the genus *Lactobacillus* lactic acid bacterium is added singly and when the fermented milk (control fermented milk) is added singly. These results suggest that the IL-10 production promoting activity is increased by a composition containing a genus *Lactobacillus* lactic acid bacterium and a fermented milk, and further, that the production of IL-10 is synergistically promoted in the composition containing a genus *Lactobacillus* lactic acid bacterium and a fermented milk, as compared to a composition containing the genus *Lactobacillus* lactic acid bacterium or the fermented milk singly.

The results in FIG. 4B have shown that, in cases where the lactic acid bacterium-containing fermented milk containing the genus *Lactobacillus* lactic acid bacterium and the fermented milk is added, the production of IL-12 is significantly inhibited as compared to the case where the genus *Lactobacillus* lactic acid bacterium is added singly, and the production of IL-12 tends to be inhibited as compared to the case where the fermented milk (control fermented milk) is added singly (p = 0.08). These results suggest that the production of IL-12 is inhibited by a composition containing a genus *Lactobacillus* lactic acid bacterium and a fermented milk.

Further, FIG. 5 shows the results of the ratio (the amount of IL-10/the amount of IL-12) of the amount of IL-10 with respect to the amount of IL-12 calculated based on the results shown in FIG. 4A and FIG. 4B.

The results in FIG. 5 have shown that the ratio of the amount of IL-10/the amount of IL-12 is significantly increased in cases where the lactic acid bacterium-containing fermented milk containing the genus *Lactobacillus* lactic acid bacterium and the fermented milk is added, as compared to the case where the genus *Lactobacillus* lactic acid bacterium or the fermented milk (control fermented milk) is added singly. Further, it has been shown that the ratio of the amount of IL-10/the amount of IL-12 when the lactic acid bacterium-containing fermented milk containing the genus *Lactobacillus* lactic acid bacterium and the fermented milk is added, is higher than the total of the respective ratios of the amount of IL-10/the amount of IL-12 when the genus *Lactobacillus* lactic acid bacterium is added singly and when the fermented milk (control fermented milk) is added singly. These results suggest that the ratio of the amount of IL-10/the amount of IL-12 is synergistically increased in a composition containing a genus *Lactobacillus* lactic acid bacterium and a fermented milk, as compared to a composition containing the genus *Lactobacillus* lactic acid bacterium or the fermented milk singly.

### (2) Effects on Cytokine Production Promoting Activity: No. 2

The lactic acid bacteria starter A and the lactic acid bacterium *Lactobacillus plantarum* strain OLL2712 which had been subjected to a heat treatment were added to a raw material milk (a mixture containing raw milk, skimmed milk powder, cream, sugar and stevia). The resulting mixture was maintained at 43°C until a lactic acid acidity (acidity) of 0.7% was reached to ferment the raw material milk, to prepare a lactic acid bacterium-containing fermented milk 1 (set yogurt). The heat treatment of the lactic acid bacterium was carried out by concentrating the bacterial cells and then heating the bacterial cells at 60°C for 10 minutes. The amount of bacterial cells of the genus *Lactobacillus* lactic acid bacterium contained in the resulting test composition 1 was 5 × 10⁹ or more cells/112 g. Further, a lactic acid bacterium-containing fermented milk 2 was prepared in the same manner as the lactic acid bacterium-containing fermented milk 1 described above, except that the genus *Lactobacillus* lactic acid bacterium to be added was changed to the lactic acid bacterium *Lactobacillus plantarum* strain P200021 which had been subjected a heat treatment. Meanwhile, a control fermented milk (set yogurt) was prepared in the same manner as the test composition described above, except that the genus *Lactobacillus* lactic acid bacterium was not added.

The amount of production of the anti-inflammatory cytokine IL-10 was measured in the same manner as in the above-described Example 1, except that each composition one day after the preparation was added to bone marrow-derived dendritic cells (10⁵ cells/well) at a concentration of 1%. Further, the amount of production of IL-12 was measured in the same manner as the measurement of the amount of production of IL-10 in the above-described Example 1, except that "Mouse IL-12 (p70) ELISA Set" (manufactured by Becton Dickinson Company) was used as the ELISA kit. The amount of production (concentration) of IL-12 as used herein was measured as the amount of production (concentration) of active IL-12 (p70). The measured results of the amount of production of IL-10 and the amount of production of IL-12 are shown in FIG. 6A and FIG. 6B, respectively.

The results in FIG. 6A have shown that the production of IL-10 is significantly promoted in cases where the lactic acid bacterium-containing fermented milk 1 or 2 containing a genus *Lactobacillus* lactic acid bacterium and a fermented milk is added, as compared to the case where the fermented milk (control fermented milk) is added singly. Further, it has been shown that the production of IL-10 is significantly promoted in cases where the lactic acid bacterium-containing fermented milk 1 is added, even compared to the case of adding the lactic acid bacterium-containing fermented milk 2. These results suggest that the IL-10 production promoting activity is markedly increased by a composition containing a genus *Lactobacillus* lactic acid bacterium and a fermented milk, and that the IL-10 production promoting activity is especially markedly increased by a composition containing the lactic acid bacterium *Lactobacillus plantarum* strain OLL2712 and a fermented milk.

The results in FIG. 6B have shown that the production of IL-12 is significantly inhibited in cases where the lactic acid bacterium-containing fermented milk 1 or 2 containing a genus *Lactobacillus* lactic acid bacterium and a fermented milk is added, as compared to the case where the fermented milk (control fermented milk) is added singly. The above-described results suggest that the production of IL-12 is markedly inhibited by a composition containing a genus *Lactobacillus* lactic acid bacterium and a fermented milk.

Further, FIG. 7 shows the results of the ratio (the amount of IL-10/the amount of IL-12) of the amount of IL-10 with respect to the amount of IL-12 calculated based on the results shown in FIG. 6A and FIG. 6B.

The results in FIG. 7 have shown that the ratio of the amount of IL-10/the amount of IL-12 is significantly increased in cases where the lactic acid bacterium-containing fermented milk 1 or 2 containing a genus *Lactobacillus* lactic acid bacterium and a fermented milk is added, as compared to the case where the fermented milk (control fermented milk) is added singly. Further, it has been shown that the ratio of the amount of IL-10/the amount of IL-12 is significantly increased in cases where the lactic acid bacterium-containing fermented milk 1 is added, even compared to the case of adding the lactic acid bacterium-containing fermented milk 2. These results suggest that the ratio of the amount of IL-10/the amount of IL-12 is markedly increased by a composition containing a genus *Lactobacillus* lactic acid bacterium and a fermented milk, and further, that the ratio of the amount of IL-10/the amount of IL-12 is especially markedly increased by a composition containing the lactic acid bacterium *Lactobacillus plantarum* strain OLL2712 and a fermented milk.

### (3) Effects on Cytokine Production Promoting Activity: No. 3

A lactic acid bacterium-containing fermented milk 1 and a lactic acid bacterium-containing fermented milk 2 as well as a control fermented milk were prepared in the same manner as in the section (2) described above, except that the lactic acid bacteria starter B was used instead of the lactic acid bacteria starter A as the starter to be used when fermenting the raw material milk to prepare a fermented milk.

The amounts of production of the anti-inflammatory cytokine IL-10 and the proinflammatory cytokine IL-12 were measured in the same manner as in the section (2) described above. The measured results of the amount of production of IL-10 and the amount of production of IL-12 are shown in FIG. 8A and FIG.8B, respectively.

The results in FIG. 8A have shown that the production of IL-10 is significantly promoted in cases where the lactic acid bacterium-containing fermented milk 1 or 2 containing a genus *Lactobacillus* lactic acid bacterium and a fermented milk is added, as compared to the case where the fermented milk (control fermented milk) is added singly. Further, it has been shown that the production of IL-10 is significantly promoted in cases where the lactic acid bacterium-containing fermented milk 1 is added, even compared to the case of adding the lactic acid bacterium-containing fermented milk 2. These results suggest that the IL-10 production promoting activity is markedly increased by a composition containing a genus *Lactobacillus* lactic acid bacterium and a fermented milk, and further, that the IL-10 production promoting activity is especially markedly increased by a composition containing the lactic acid bacterium *Lactobacillus plantarum* strain OLL2712 and a fermented milk.

The results in FIG. 8B have shown that the production of IL-12 is markedly inhibited in cases where the lactic acid bacterium-containing fermented milk 1 or 2 containing a genus *Lactobacillus* lactic acid bacterium and a fermented milk is added, as compared to the case where the fermented milk (control fermented milk) is added singly. The above-described results suggest that the production of IL-12 is markedly inhibited by a composition containing a genus *Lactobacillus* lactic acid bacterium and a fermented milk.

Further, FIG. 9 shows the results of the ratio (the amount of IL-10/the amount of IL-12) of the amount of IL-10 with respect to the amount of IL-12 calculated based on the results shown in FIG. 8A and FIG. 8B.

The results in FIG. 9 have shown that the ratio of the amount of IL-10/the amount of IL-12 is significantly increased in cases where the lactic acid bacterium-containing fermented milk 1 or 2 containing a genus *Lactobacillus* lactic acid bacterium and a fermented milk is added, as compared to the case where the fermented milk (control fermented milk) is added singly. The above-described results suggest that the ratio of the amount of IL-10/the amount of IL-12 is markedly increased by a composition containing a genus *Lactobacillus* lactic acid bacterium and a fermented milk. Based on the above, it has been shown that the combined use of a genus *Lactobacillus* lactic acid bacterium and a fermented milk leads to an increase in the IL-10 production promoting activity, a marked inhibition of the production of IL-12 and a marked increase in the ratio of the amount of IL-10/the amount of IL-12, as compared to using the genus *Lactobacillus* lactic acid bacterium singly or the fermented milk singly, regardless of the type of the starter used in the production of the fermented milk.

### INDUSTRIAL APPLICABILITY

According to the present invention, the production of IL-10 in a subject can be promoted. Further, according to the present invention, the production of IL-12 in a subject can be inhibited. Since IL-10 is known to inhibit inflammation and IL-12 is known to promote inflammation, the present invention enables to ameliorate inflammation, a disease caused by inflammation, and the like.

## Claims

1. A composition for promoting the production of interleukin-10, the composition comprising: a lactic acid bacterium belonging to the genus *Lactobacillus;* and a fermented milk.

2. The composition according to claim 1, wherein the composition is for inhibiting the production of interleukin-12.

3. The composition according to claim 1 or 2, wherein the composition is for increasing the ratio of the amount of interleukin-10 with respect to the amount of interleukin-12.

4. The composition according to any one of claims 1 to 3, wherein the composition is an anti-inflammatory composition.

5. The composition according to any one of claims 1 to 4, wherein the lactic acid bacterium is *Lactobacillus plantarum.*

6. The composition according to any one of claims 1 to 5, wherein the lactic acid bacterium contains a dead bacterial cell of the lactic acid bacterium.

7. The composition according to any one of claims 1 to 6, wherein the lactic acid bacterium contains a heat-treated dead bacterial cell.

8. The composition according to any one of claims 1 to 7, wherein the composition is a food composition.

9. The composition according to any one of claims 1 to 7, wherein the composition is a pharmaceutical composition.

10. The composition according to any one of claims 1 to 9, wherein the lactic acid bacterium has a 16S rRNA gene having a homology of 90% or more to the nucleotide sequence represented by SEQ ID NO: 1.

11. The composition according to any one of claims 1 to 10, wherein the lactic acid bacterium is *Lactobacillus plantarum* strain OLL2712 deposited under the accession number FERM BP-11262.

12. The composition according to any one of claims 1 to 11, wherein the fermented milk has an average particle size of from 0.5 to 100 µm.

13. A method of producing the composition according to any one of claims 1 to 12, the production method comprising:
a starter inoculation step of inoculating a raw material milk with a starter to obtain a fermented milk base material;
a fermentation step of fermenting the fermented milk base material to obtain a fermented milk;
a lactic acid bacterium addition step of adding a lactic acid bacterium belonging to the genus *Lactobacillus* to at least one selected from the group consisting of the raw material milk, the fermented milk base material and the fermented milk, to obtain a lactic acid bacterium-containing composition; and
a stirring step of stirring at least one selected from the group consisting of the raw material milk, the fermented milk base material, the fermented milk and the lactic acid bacterium-containing composition.

14. The production method according to claim 13, wherein the stirring in the stirring step is carried out under such conditions that the average particle size of the fermented milk containing the lactic acid bacterium belonging to the genus *Lactobacillus* is adjusted within the range of from 0.5 to 100 µm.

15. The production method according to claim 13 or 14, wherein the stirring in the stirring step is carried out under the condition of from 30 to 500 rpm.

16. A use of the composition according to any one of claims 1 to 12 for the production of a pharmaceutical product for treating a disease caused by a decrease in the amount of production of interleukin-10.

17. A use of the composition according to any one of claims 1 to 12 for the production of a food or beverage product for treating a disease caused by a decrease in the amount of production of interleukin-10.

18. A use of the composition according to any one of claims 1 to 12 for promoting the production of interleukin-10.

19. A method of promoting the production of interleukin-10, the method comprising allowing a subject in need thereof to ingest an effective amount of the composition according to any one of claims 1 to 12.
